# FASCICULE DE BREVET EUROPEEN

(11) **EP 2 307 436 B1**
(45) Date de publication et mention de la délivrance du brevet: **23.11.2011**
(21) Numéro de dépôt: 09772756.4
(22) Date de dépôt: 03.07.2009
(51) Int. Cl.: C07H 21/00, C12Q 1/68, G01N 33/52, G01N 33/58

(54) **NOUVELLE SONDE DE DÉTECTION**
NEUE NACHWEISSONDE
NOVEL DETECTION PROBE

(30) Priorité: 04.07.2008 FR 0854549
(43) Date de publication de la demande: 13.04.2011
(73) Titulaire: Biomérieux, 69280 Marcy L'etoile (FR)
(72) Inventeur: Laayoun, Ali, 38260 La Frette (FR); Bernal Mendez, Eloy, F-38070 Saint Quentin Fallavier (FR)
(86) Numéro de dépôt international: PCT/FR2009/051315
(87) Numéro de publication internationale: WO 2010/001074

(56) Documents cités:
- ZHANG ET AL: "Amplification of circularizable probes for the detection of target nucleic acids and proteins" CLINICA CHIMICA ACTA, ELSEVIER BV, AMSTERDAM, NL, vol. 363, no. 1-2, 1 janvier 2006 (2006-01-01), pages 61-70, XP005194901 ISSN: 0009-8981
- ZHANG D Y ET AL: "DETECTION OF TARGET NUCLEIC ACIDS AND PROTEINS BY AMPLIFICATION OF CIRCULARIZABLE PROBES" EXPERT REVIEW OF MOLECULAR DIAGNOSTICS, FUTURE DRUGS, LONDON, GB, vol. 3, no. 2, 1 janvier 2003 (2003-01-01) , pages 237-248, XP008061300 ISSN: 1473-7159
- YI JIZU ET AL: "Molecular Zipper: a fluorescent probe for real-time isothermal DNA amplification" NUCLEIC ACIDS RESEARCH, OXFORD UNIVERSITY PRESS, SURREY, GB, vol. 34, no. 11, 1 janvier 2006 (2006-01-01), pages E81-E85, XP002434420 ISSN: 0305-1048

## Description

La présente invention concerne la détection d'acides nucléiques par de nouvelles sondes nucléotidiques. L'invention trouve son application dans le domaine du diagnostic.

Aujourd'hui, la recherche de séquences nucléotidiques cibles représente un objectif majeur dans de nombreux laboratoires et principalement dans le domaine médical ou agroalimentaire. Dans ces domaines, la recherche de séquences cibles vise par exemple la détection d'organismes pathogènes, la recherche d'une contamination bactérienne dans une chaîne agroalimentaire ou encore le diagnostic de mutations à l'origine de maladies génétiques ou de cancers. Les difficultés majeures de ces méthodes résident dans la spécificité, la sensibilité, la rapidité et la reproductibilité du test utilisé. Il est indispensable de pouvoir donner un résultat certain et ceci essentiellement dans le domaine du diagnostic.

Différents types de méthodes sont décrits dans la littérature. Elles reposent généralement sur les propriétés d'appariement des brins complémentaires d'acides nucléiques, communément appelée «hybridation d'acides nucléiques» ou simplement «hybridation». cf. D.ZHANG et al., Clinica chimica acta 363 (2006) pp 61-70, or J. Yi, Nucleic acid research (2006) Vol 34 (11), l81. D'une manière générale, après avoir déterminé la séquence spécifique d'un organisme ou d'une maladie qui doit être analysée, il convient d'extraire les acides nucléiques d'un échantillon, et éventuellement d'amplifier et de détecter la séquence d'intérêt. Ainsi de nombreuses méthodes d'amplification ont été développées telles que PCR, LCR, NASBA, TMA, SDA. De même un grand nombre de techniques de détection existe. L'une d'elles met en oeuvre des sondes particulières, décrites par Tyagi & Kramer (Nature Biotech, 1996, 14 :303-308), communément connues sous le nom de « molecular beacons ». Ces sondes ont une structure en épingle à cheveux comprenant une partie « boucle » simple brin et une partie « tige » double brin. La partie « boucle » contient une séquence complémentaire de la séquence nucléotidique cible et la partie « tige » est formée par deux séquences complémentaires l'une de l'autre. L'extrémité libre d'une des séquences complémentaires de la partie « tige » est marquée par un fluorophore tandis que l'autre extrémité libre de l'autre brin de la partie « tige » est couplée à un extincteur de fluorescence ou « quencher ». En l'absence d'une séquence cible complémentaire, la sonde de détection est dans une forme en épingle à cheveux et la sonde ne produit pas de fluorescence, l'énergie du fluorophore étant transférée directement à l'extincteur de fluorescence. Lorsque cette sonde s'hybride à une séquence cible, elle perd sa configuration, les extrémités 5' et 3' s'éloignent et l'extincteur de fluorescence et le fluorophore sont séparés l'un de l'autre. La fluorescence émise reflète alors l'hybridation de la sonde sur la cible qui est détectée pendant son amplification et éventuellement quantifiée. On parle alors de la détection en phase homogène en temps réel (ou détection en « real-time »). Toutefois, ces sondes molecular beacon peuvent présenter une ouverture prématurée non désirée de la partie « tige » en présence de certains contaminants. En effet, il a été observé que les sondes molecular beacons interagissaient, par des mécanismes inconnus, avec les enzymes présentent dans le milieu réactionnel tel que celui utilisé dans une amplification de type NASBA par exemple. Ces interactions se produisent en l'absence même de toute cible biologique et sont à l'origine d'une augmentation de signal de fluorescence non spécifique.

Les sondes molecular beacons présentent des contraintes de synthèse importantes nécessitant un design très particulier au niveau des séquences de la tige. En effet, elles peuvent s'hybrider de façon non spécifique avec la cible à détecter, notamment lorsque celle-ci est riche en bases GC, induisant des faux positifs ou des bruits de fond importants qui affectent la sensibilité de détection. De plus, il est nécessaire d'introduire deux séquences complémentaires pour former la tige, la séquence complète de la sonde en épingle à cheveux comprend alors au moins 30 nucléotides, alors que seulement 20 nucléotides sont généralement nécessaires pour la reconnaissance moléculaire. De ce fait, le rendement de synthèse de ces sondes en épingle à cheveux est assez limité. Un autre inconvénient de ces sondes est la difficulté de design de la structure en épingle à cheveux du fait de la structure « tige-boucle » qui facilite le repliement de la boucle sur elle même et les interactions internes « boucle-boucle ». Ainsi, plusieurs designs et synthèses sont alors souvent nécessaires avant de trouver la séquence qui possède les caractéristiques thermodynamiques recherchées.

Différentes solutions ont été proposées pour tenter de pallier au problème d'hybridation non spécifique de la tige. Ainsi Crey-Desbiolles et al. (Nucleic Acids Res., May 2005; 33: e77) ont proposé la substitution des nucléotides naturels de la tige par des nucléotides modifiés qui s'hybrident entre eux pour former la tige mais qui ne peuvent pas s'hybrider avec les nucléotides naturels de la cible. L'inconvénient de cette approche est principalement le besoin d'introduire, à chaque extrémité de la séquence de reconnaissance, au moins quatre à cinq unités de ce nucléotide modifié, alors qu'il n'est pas commercialement disponible, ce qui complique la synthèse de la sonde en épingle à cheveux. D'autre part, cette approche ne résout pas le problème du nombre élevé de nucléotides de la séquence complète ni celui de la difficulté de design de la structure de l'épingle à cheveux.

Une autre solution proposée par Browne (J. Am. Chem. Soc., 2005, 127, 1989-1994) consiste à introduire des nucléotides inversés dans les deux séquences de la tige. Dans ce mode de réalisation, les séquences de la tige ne peuvent pas s'hybrider directement sur les séquences de la cible attenantes à la séquence hybridée avec la boucle, à cause de leur orientation parallèle avec la cible. L'inconvénient de cette technique est que les séquences de la tige peuvent interagir avec d'autres séquences de la cible plus lointaines qui pourraient se replier et se rendre accessibles pour cette interaction. De plus, les problèmes du nombre élevé de nucléotides de la séquence complète et celui de l'instabilité de la structure en épingle à cheveux ne sont pas résolus par cette approche.

Une autre solution proposée par Tsourkas et al. (Nucleic Acids Res., Oct 2002; 30: 4208 - 4215.) consiste à designer la sonde en épingle à cheveux de telle façon que l'une des séquences formant la tige fait aussi partie de la séquence de reconnaissance. Cette solution permet de raccourcir la séquence complète de la sonde. Cependant, une perte de spécificité, associée à ce design, a été observée. De plus, pour la même séquence de reconnaissance, ce type de design possède une boucle plus courte, ce qui restreint sa flexibilité et renforce les interactions boucle-boucle, rendant les caractéristiques thermodynamiques de la sonde moins prévisibles.

Il existe donc un réel besoin pour un nouveau format de sonde permettant de pallier les inconvénients de l'art antérieur.

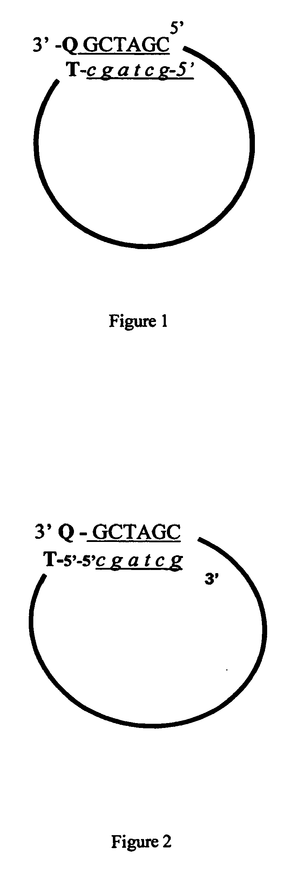

A ce titre, l'invention concerne une nouvelle sonde de détection d'un acide nucléique cible, constituée d'un brin nucléotidique marqué comportant trois fragments :
- un premier fragment présentant une première séquence de fermeture,
- un deuxième fragment dont tout ou partie présente une séquence de reconnaissance destinée à la reconnaissance moléculaire de l'acide nucléique cible,
- un troisième fragment présentant une deuxième séquence de fermeture, et
- au moins deux marqueurs, une des extrémités du brin de ladite sonde de détection étant libre de tout marqueur,
dans laquelle, lorsque les deux séquences de fermeture sont hybridées ensemble, la sonde de détection a une forme complètement circulaire, lesdites séquences de fermeture maintenant ainsi ladite sonde dans une conformation non détectable en l'absence dudit acide nucléique cible. On entend par complètement circulaire, une conformation de la sonde lorsque les deux séquences de fermeture sont hybridées l'une à l'autre pour former un duplex, l'une des extrémités de l'une des séquences de fermeture étant reliée à l'extrémité opposée de l'autre séquence de fermeture par l'intermédiaire de la séquence de reconnaissance, l'ensemble formant un cercle selon les figures 1 et 2. A l'inverse d'une structure en épingle à cheveux cette conformation permet de maintenir la séquence boucle en disposition linéaire, étirée entre les deux extrémités de la tige, ce qui empêche les interactions internes dans la séquence de la boucle, et facilite aussi son accessibilité par la cible complémentaire.

Dans un mode de réalisation intéressant de l'invention, la sonde de détection est caractérisée en ce qu'un premier marqueur est porté par un nucléotide du premier fragment et un second marqueur est porté par un nucléotide du deuxième ou du troisième fragment, les deux nucléotides étant avoisinants lorsque les deux séquences de fermeture sont hybridées.

Selon un mode préféré de réalisation de l'invention, la sonde de détection est caractérisée en ce que tout ou partie des deux séquences de fermeture s'hybrident de façon parallèle. De façon avantageuse, l'une des séquences de fermeture est une séquence constituée de nucléotides d'anomérie *alpha.*

Selon un autre mode préféré de réalisation de l'invention, la sonde de détection est caractérisée en ce que tout ou partie des deux séquences de fermeture s'hybrident de façon anti-parallèle. De façon avantageuse une des deux séquences de fermeture est liée à la séquence de reconnaissance par une liaison inversée 5'-5' ou 3'-3'.

De façon avantageuse, au moins un marqueur est un fluorophore et au moins un autre marqueur est un extincteur de fluorescence. Ainsi, lorsque les deux séquences de fermeture sont hybridées ensemble, les nucléotides portant les marqueurs sont avoisinants de telle sorte que toute ou une grande partie de l'énergie du fluorophore après excitation est transférée à l'extincteur de fluorescence. De préférence, les deux marqueurs sont portés par des nucléotides dont la position est l'un en face de l'autre, ces deux nucléotides étant reliés l'un à l'autre par des liaisons hydrogène, formant un appariement de bases, ou distants de 1 à 5 nucléotides.

De façon avantageuse, tout ou partie de la première ou de la seconde séquence de fermeture peut s'hybrider sur la séquence cible. Cette conformation permet un meilleur contrôle de la distance entre le fluorophore et l'extincteur de fluorescence, car une des séquences de fermeture étant immobilisée sur la cible, les deux séquences de fermeture ne peuvent pas s'approcher entre elles autant que si elles étaient toutes les deux en conformation flexible simple brin. Cette conformation a aussi l'avantage de diminuer le nombre de nucléotides libres pouvant interagir de façon non spécifique et celle de diminuer le nombre de nucléotides de la séquence complète. De façon avantageuse, la longueur de la sonde est comprise entre 25 et 40 nucléotides.

Selon le mode de réalisation précédent, la sonde une fois hybridée à la séquence cible, a une séquence de fermeture qui participe à l'hybridation tandis que l'autre séquence de fermeture est dans l'incapacité de s'hybrider du fait de son orientation inversée ou de la présence de nucléotides *alpha.* Cette conformation permet de limiter les hybridations non spécifiques. De plus, au cours du design de la sonde, l'ajout de la séquence de fermeture modifiée (*alpha* ou inversée) au reste de la sonde, n'influence pas le design de la séquence de reconnaissance. Ainsi, le design se limite à définir la bonne séquence complémentaire de la cible en fonction des conditions de détection, et ajouter après à l'une de ses extrémités une séquence de fermeture modifiée (*alpha* ou inversée) complémentaire de l'autre séquence de fermeture capable de s'hybrider à la cible, sans devoir vérifier les interactions possibles avec la cible ni devoir ajouter une séquence à chaque extrémité, comme c'est le cas pour les sondes en épingle à cheveux. Le design de la sonde en est donc facilité.

Encore de façon avantageuse, la longueur des séquences de fermeture est comprise entre 6 et 30 nucléotides, préférentiellement entre 6 et 15 nucléotides.

Dans un mode particulier, une séquence nucléotidique est attachée à la sonde de détection. De préférence, elle est attachée à l'extrémité de la séquence de fermeture naturelle c'est à dire à la séquence qui n'est ni inversée ni constituée de nucléotides *alpha.* Ainsi la sonde de détection selon la présente invention peut être adaptée au design de la sonde appelée « tentacle probe » selon la demande de brevet WO-A-07/114986.

La sonde de détection selon la présente invention peut être mise en oeuvre dans n'importe quel test où une sonde nucléotidique est utilisée pour détecter un acide nucléique cible. Ainsi, un autre objet de la présente invention porte sur une méthode pour détecter une séquence cible susceptible d'être présente dans un échantillon comprenant les étapes suivantes :
- mettre en contact l'échantillon avec une sonde de détection selon la présente invention,
- détecter la formation d'un hybride entre la sonde de détection et la séquence cible, indiquant la présence de ladite séquence cible dans ledit échantillon.

Dans un mode préféré de l'invention, la détection se fait conjointement à une réaction d'amplification de la séquence cible, on parle alors de détection en temps réel. Dans un autre mode préféré, la détection se fait après une réaction d'amplification de la séquence cible, on parle alors de détection « end-point ». La réaction d'amplification peut être basée sur n'importe quelle méthode d'amplification, linéaire ou exponentielle, telle que PCR, LCR, NASBA, TMA, SDA, RCA. Préférentiellement la méthode d'amplification est une méthode d'amplification produisant des acides nucléiques simple brin ADN ou ARN, telle que la NASBA, la TMA ou la PCR asymétrique.

Un autre objet de l'invention porte sur un kit pour détecter une séquence cible comprenant au moins une sonde de détection selon la présente invention. Ce kit peut éventuellement contenir les réactifs et séquences amorces nécessaires à la réalisation d'une réaction d'amplification. Par exemple, un kit selon la présente invention, adapté à une amplification NASBA, peut contenir des nucléotides et des enzymes d'amplification telles que les transcriptases inverses, la RNase H et la T7 ARN polymérase.

Les définitions suivantes permettront de mieux comprendre l'invention.

Le terme « acide nucléique » ou « séquence nucléotidique » signifie un enchaînement d'au moins deux désoxyribonucléotides ou ribonucléotides comprenant éventuellement au moins un nucléotide modifié, par exemple au moins un nucléotide comportant une base nucléique modifiée, telle que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant l'hybridation. Ce polynucléotide peut aussi être modifié au niveau de la liaison internucléotidique comme par exemple les phosphorothioates, les H-phosphonates, les alkyl-phosphonates, au niveau du squelette comme par exemple les *alpha-*oligonucléotides (FR 2 607 507) ou les PNA (M. Egholm et al., J. Am. Chem. Soc., 114, 1895-1897, 1992) ou les 2' O-alkyl ribose et les LNA (BW, Sun et al., Biochemistry, 4160-4169, 43, 2004). L'acide nucléique peut être naturel ou synthétique, un oligonucléotide, un polynucléotide, un fragment d'acide nucléique, un ARN ribosomique, un ARN messager, un ARN de transfert, un acide nucléique obtenu par une technique d'amplification enzymatique, telle que :
- PCR (Polymerase Chain Reaction), décrite dans les brevets US-A-4,683,195, US-A-4,683,202 et US-A-4,800,159, et sa dérivée RT-PCR (Reverse Transcription PCR), notamment dans un format en une étape, tel que décrit dans le brevet EP-B-0.569.272,
- LCR (Ligase Chain Reaction), exposée par exemple dans la demande de brevet EP-A-0.201.184,
- RCR (Repair Chain Reaction), décrite dans la demande de brevet WO-A-90/01069,
- 3SR (Self Sustained Sequence Replication) avec la demande de brevet WO-A-90/06995,
- NASBA (Nucleic Acid Sequence-Based Amplification) avec la demande de brevet WO-A-91/02818,
- TMA (Transcription Mediated Amplification) avec le brevet US-A-5,399,491, et
- RCA (Rolling Circle Amplification) décrite dans le brevet US-6,576,448.

Au sens de la présente invention, on entend par « cible » ou « acide nucléique cible », une séquence nucléotidique dont au moins une partie de l'enchaînement en motifs nucléotidiques est spécifique et complémentaire de la séquence nucléotidique de la sonde de détection utilisée. La cible peut être naturelle ou issue d'une réaction d'amplification enzymatique *in vitro,* comme la NASBA (Nucleic Acid Sequence-Based Amplification), la PCR (Polymerase Chain Reaction) ou toute autre technique connue de l'homme du métier. Lorsque la séquence cible est issue d'une réaction d'amplification enzymatique *in vitro,* les séquences produites par cette amplification sont appelées des « amplicons ».

Par « sonde de détection », on entend une séquence nucléique de 10 à 100 motifs nucléotidiques, notamment de 10 à 50 motifs nucléotidiques et préférentiellement de 25 à 40 nucléotides. Cette sonde comprend un fragment nucléotidique dit deuxième fragment dont au moins une partie de l'enchaînement en motifs nucléotidiques est spécifique et complémentaire de la séquence nucléotidique de la cible. Cette sonde comprend également deux autres fragments dit premier et troisième fragments présentant chacun une séquence de fermeture dont la longueur est comprise entre 6 et 30 nucléotides et préférentiellement entre 6 et 15 nucléotides.

Par « premier fragment », on entend une séquence nucléotidique qui peut être complémentaire et de polarité adaptée audit troisième segment lors de la présence de ce dernier.

Par « deuxième fragment », on entend une séquence nucléotidique complémentaire et de polarité adaptée à la séquence de la cible.

Par « troisième fragment », on entend une séquence nucléotidique complémentaire et de polarité adaptée audit premier segment.

Par « marqueur », on entend une molécule portée par un nucléotide. Le lien entre le marqueur et le nucléotide peut se faire de différentes façons connues de l'homme du métier. Le couplage manuel se fait par l'utilisation de marqueurs portant un groupement activé, typiquement un carboxyle ou un thiol, qui sont couplés sur un nucléotide interne modifié portant le groupement réactif correspondant (amine ou thiol, par exemple), ou sur une extrémité du brin nucléotidique modifiée avec ces mêmes groupements réactifs. Le couplage automatique se fait par l'utilisation de phosphoramidites portant le marqueur, et alors le couplage se fait pendant la synthèse automatisée du brin nucléotidique, soit sur une extrémité du brin, soit sur une position interne, en fonction du type de phosphoramidite utilisé.

Par « fluorophore », on entend une molécule qui émet un signal de fluorescence quand elle est excitée par de la lumière à une longueur d'onde qui convient. Le fluorophore peut être notamment une rhodamine ou un dérivé tel que Texas Red, une fluorescéine ou un dérivé, un fluorophore de la famille des Alexa tels que l'Alexa532 et l'Alexa647, Alexa 405, Alexa 700, Alexa 680, ou tout autre fluorophore qui convienne en fonction de l'appareil de mesure utilisé. Les fluorophores disponibles pour les sondes de détection sont très variés et connus de l'homme du métier.

Au sens de la présente invention, on entend par « fluorescéine » une molécule chimique aromatique qui émet un signal de fluorescence avec un maximum d'émission autour de 530 nm, quand elle est excitée par de la lumière à une longueur d'onde autour de 495 nm.

Par « extincteur de fluorescence » ou « quencher » ou « quencheur », on entend une molécule qui interfère avec la fluorescence émise par un fluorophore. Cet extincteur peut être choisi parmi des molécules aromatiques non fluorescentes, pour éviter des émissions parasites. Préférentiellement, le dit extincteur est un Dabsyl ou un Dabcyl ou un « Black hole quencher™» qui sont des molécules aromatiques non fluorescentes qui empêchent l'émission de fluorescence quand elles se trouvent physiquement à proximité d'un fluorophore. La technique de transfert d'énergie de fluorescence par résonance (FRET) peut également être utilisé tel que décrit par exemple dans Fluorescent Energy Transfer Nucleic Acid Probes, p. 4, Ed. V.V. Didenko, Humana Press 2006, ISSN 1064-3745. Le quencheur peut également être choisi parmi des molécules fluorescentes telles que par exemple la TAMRA (carboxytetramethylrhodamine).

Par « polarité », on entend l'orientation de la séquence nucléotidique, 5' vers 3' ou 3' vers 5', par rapport à sa séquence complémentaire. Ainsi des segments peuvent être orientés de façon :
□ antiparallèle : dans ce cas l'oligonucléotide est hybridé dans le sens inversé de la séquence complémentaire. Dans la présente invention, un des modes de réalisation présente une des séquences de fermeture qui est alors constituée de nucléotides inversés afin que la sonde de détection ait une forme circulaire lorsque ladite séquence de fermeture est hybridée à l'autre séquence de fermeture.
□ parallèle : dans ce cas, l'oligonucléotide est hybridé dans le même sens que la séquence complémentaire. Dans la présente invention, un des modes de réalisation présente une des séquences de fermeture qui est alors constituée de nucléotides *alpha* afin que la sonde de détection ait une forme circulaire lorsque ladite séquence de fermeture est hybridée à l'autre séquence de fermeture.

Par « hybridation », on entend le processus au cours duquel, dans des conditions appropriées, deux fragments nucléotidiques simple brin, ayant en tout ou partie des séquences suffisamment complémentaires, sont susceptibles de former un double brin ou « duplex » stabilisé par des liaisons hydrogènes entre les bases nucléiques. Les conditions d'hybridation sont déterminées par la stringence, c'est-à-dire la rigueur et la faible salinité des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier.

Par « nucléotides *alpha* » ou « nucléotide d'anomérie *alpha* », on entend des désoxyribonucléotides ou des ribonucléotides à configuration anomérique non naturelle *alpha,* dans lequel la base azotée portée par le carbone anomérique du désoxyribose est située en dessous du plan au lieu d'être en dessus du plan comme dans le cas des nucléotides *beta*. La base azotée des nucléotides *alpha* peut être une base nucléique modifiée, telle que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant l'hybridation. Les nucléotides *alpha* peuvent aussi être modifiés au niveau de la liaison internucléotidique comme par exemple les phosphorothioates, les H-phosphonates, les alkyl-phosphonates ou au niveau du squelette comme par exemple les 2' O-alkyl ribose, les PNA et les LNA. Préférentiellement, les nucléotides *alpha* sont ceux décrits dans la demande WO-A-88/04301.

Par « nucléotide inversé » on entend des désoxyribonucléotides ou des ribonucléotides ayant une orientation inverse à celle de la séquence qui les contient. Classiquement, la synthèse se faisant dans le sens 3' vers 5', les nucléotides inversés sont introduits à la suite ou avant le reste du brin nucléotidique, dans le sens 5' vers 3'. La jonction entre les nucléotides inversés et le reste du brin se fait alors par une liaison 5'-5' ou 3'-3'. On pourra se référer à Koga M. et al., J. Org. Chem., 1991, 56, 3757. Les nucléotides inversés peuvent comprendre éventuellement au moins un nucléotide modifié, par exemple au moins un nucléotide comportant une base nucléique modifiée, telle que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant hybridation. Les nucléotides inversés peuvent aussi être modifiés au niveau du squelette comme par exemple les PNA les 2' O-alkyl ribose et les LNA.

Par « extrémité », on entend le point de départ et le point de terminaison de la synthèse d'un oligonucléotide généralement définis par le numéro porté par les hydroxyles libres portés par le premier ou le dernier nucléoside soit 3' ou 5'. Il est entendu que par un choix approprié des unités d'élongation (les phosphoramidites des nucléosides *alpha* ou *beta,* inversés ou non), on peut synthétiser un oligonucléotide dans le sens 3' vers 5' ou l'inverse, voire, alterner pendant la synthèse le sens de l'élongation. Ceci conduit à des oligonucléotides portant des extrémités 3'-5', 5'-3', 3'-3'ou encore 5'-5'.

Au sens de la présente invention, on entend par « échantillon biologique », tout échantillon susceptible de contenir des acides nucléiques. Ces derniers peuvent être extraits de tissus, de sang, de sérum, de salive, de cellules circulantes d'un patient ou provenir d'un produit alimentaire, agroalimentaire ou encore être d'origine environnementale. L'extraction se fait par tout protocole connu de l'homme du métier, par exemple selon le procédé d'isolation décrit dans le brevet EP-B-0.369.063.

Les figures ci-jointes sont données à titre d'exemple explicatif et n'ont aucun caractère limitatif. Elles permettront de mieux comprendre l'invention.
Figure 1 : Exemple de conformations complètement circulaires de la sonde selon la présente invention.
   **T** : dT-FAM, fluorophore
   Nucléotide en minuscule : nucléotides *alpha*
   **Q** : extincteur de fluorescence
Figure 2 : Exemple de conformations complètement circulaires de la sonde selon la présente invention.
   **T** : dT-FAM, fluorophore
   **Q** : extincteur de fluorescence
   Nucléotide en minuscule : nucléotides inversés
Figure 3 : Expérience de dénaturation thermique de la sonde selon l'invention de type *alpha* du modèle influenza B, comme décrit dans l'exemple 1 (émission de fluorescence en unité arbitraire (RFU pour Related Fluorescence Unit) en fonction de la température mesurée en degré Celsius (°C)).
Figure 3a : sonde en présence de sa cible synthétique.
Figure 3b : sonde seule sans sa cible.
Figure 4 : Expérience de dénaturation thermique de la sonde selon l'invention de type *alpha* du modèle HIV, comme décrit dans l'exemple 1 (émission de fluorescence en unité arbitraire (RFU) en fonction de la température (°C)).
Figure 4a : sonde en présence de sa cible synthétique.
Figure 4b : sonde seule sans sa cible.
Figure 5 : Expérience de dénaturation thermique de la sonde selon l'invention de type inversé du modèle influenza B, comme décrit dans l'exemple 2 (émission de fluorescence en unité arbitraire (RFU) en fonction de la température (°C).
Figure 5a : sonde en présence de sa cible synthétique.
Figure 5b : sonde seule sans sa cible.
Figure 6 : Expérience de dénaturation thermique de la sonde selon l'invention de type inversé du modèle HIV, comme décrit dans l'exemple 2 (émission de fluorescence en unité arbitraire (RFU) en fonction de la température température (°C).
Figure 6a : sonde en présence de sa cible synthétique.
Figure 6b : sonde seule sans sa cible.
Figure 7 : Expérience d'étude de la spécificité d'hybridation de la sonde de type *alpha* selon l'invention sur le modèle cible influenza B. En ordonnée est représentée la valeur de la température de fusion mesurée en degré Celsius (Tm pour « melting température ») en fonction du nombre de nucléotides complémentaires (abscisse) soit de l'un des brins de la partie « tige » du molecular beacon CpinfB (symbole carré) soit d'une des séquence de fermeture de la sonde selon l'invention Opa6d (symbole rond).
Figure 8 : Expérience d'étude de la spécificité d'hybridation de la sonde de type *alpha* selon l'invention sur le modèle cible HIV-1A. En ordonnée est représentée la valeur de la température de fusion (Tm) mesurée en degré Celsius en fonction du nombre de nucléotides complémentaires (abscisse) soit de l'un des brins de la partie « tige » du molecular beacon HIV-1A (symbole carré) soit d'une des séquence de fermeture de la sonde selon l'invention Hoa8 (symbole rond).
Figure 9 : Expérience d'étude de la spécificité d'hybridation de la sonde selon l'invention de type inversé sur le modèle cible influenza B. En ordonnée est représentée la valeur de la température de fusion (Tm) mesurée en degré Celsius en fonction du nombre de nucléotides complémentaires (abscisse) soit de l'un des brins de la partie « tige » du molecular beacon CpinfB (symbole carré) soit d'une des séquence de fermeture de la sonde selon l'invention Opi6d (symbole rond).
Figure 10 : Expérience d'étude de la spécificité d'hybridation de la sonde selon l'invention de type inversé sur le modèle HIV-1A. En ordonnée est représentée la valeur de la température de fusion (Tm) mesurée en degré Celsius en fonction du nombre de nucléotides complémentaires (abscisse) soit de l'un des brins de la partie « tige » du molecular beacon HTV-1A (symbole carré) soit d'une des séquence de fermeture de la sonde selon l'invention Hoi8 (symbole rond).
Figure 11 : Etude de l'ouverture prématurée et non désirée des sondes molecular beacon et des sondes selon l'invention en présence de contaminants dans le milieu réactionnel.
Figure 11a : Mesure du signal de fluorescence des sondes (unité arbitraire, fluorescence normalisée en divisant chaque valeur par la valeur minimale initiale en fonction du temps (exprimé en minutes) en présence d'enzyme T7 ARN polymérase.
Figure 11b : Mesure du signal de fluorescence des sondes (unité arbitraire, fluorescence normalisée en divisant chaque valeur par la valeur minimale initiale en fonction du temps (exprimé en minutes) en absence d'enzyme T7 ARN polymérase.
Figure 12 : Représentation des différentes structures utilisées dans les sondes selon l'invention.
Figure 12a : Structures des différents nucléotides utilisés : Nucléotide 1 = nucléotide d'anomérie *beta,* Nucléotide 2 = nucléotide d'anomérie *alpha,* Nucléotide 3 = nucléotide inversé.

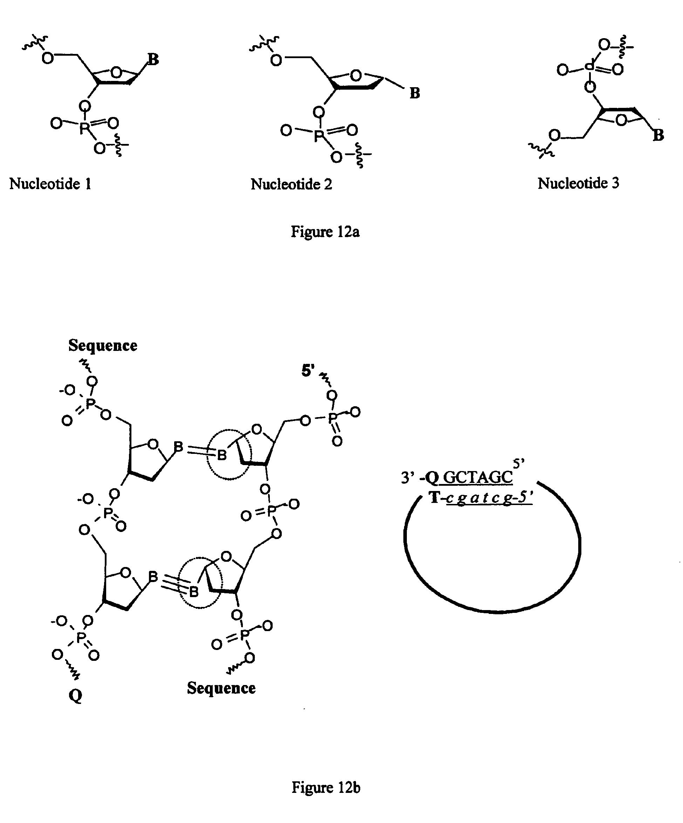

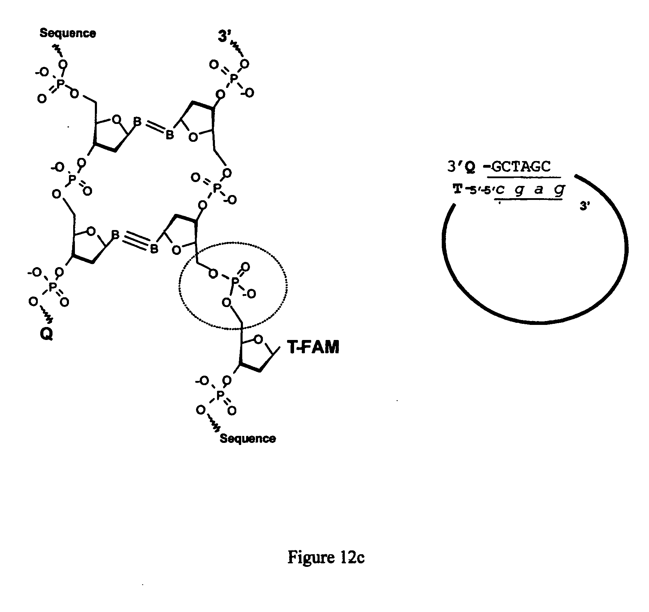

Figure 12b : Exemple d'hybridation d'une séquence de fermeture des sondes selon l'invention lorsqu'un des brins est constitué de nucléotides *alpha* avec : **T** est dT-FAM soit le fluorophore de cette molécule ; **Q** correspond à l'extincteur de fluorescence ; les nucléotides en minuscule sont des nucléotides *alpha* sur la partie droite de cette figure, alors que le cercle en pointillé met en évidence ces mêmes nucléotides d'anomérie *alpha* sur la partie gauche de cette figure ; enfin ; les nucléotides en majuscule, sur la partie droite de cette figure, sont des nucléotides *beta*, qui, sur la partie gauche, contiennent des bases B qui ne sont par encerclées.
Figure 12c : Exemple d'hybridation d'une séquence de fermeture des sondes selon l'invention lorsqu'un des brins est constitué de nucléotides inversés avec : **T** : dT-FAM, fluorophore ; **Q** : extincteur de fluorescence ; nucléotide en minuscule : nucléotides inversés ; le cercle en pointillé met en évidence la liaison 5'-5' ;

Les sondes selon l'invention, les sondes molecular beacons et les acides nucléiques cibles, utilisés dans l'ensemble des exemples ci-dessous, sont synthétisés suivant la procédure de synthèse aux phosphoramidites, connue de l'homme du métier. La méthode de synthèse aux phosphoramidites a été décrite par Beaucage et Lyer, (Tetrahedron, 48, 223-2311, 1992). Les réactifs et phosphoramidites sont achetés dans le commerce, notamment chez Eurogentec S.A. (Seraing, Belgique) et Glen Research (Sterling, Virginia, USA). Les phosphoramidites *alpha,* utilisés pour introduire les nucléotides modifiés *alpha,* sont achetés chez Chemgenes (Wilmington, MA, USA, n° catalogue ANP-1651, ANP-1652, ANP-1653, ANP-1654). Les phosphoramidites inversés, utilisés pour introduire les nucléotides inversés, sont achetés chez Glen Research (Sterling, Virginia, USA, n° catalogue 10-0001, 10-0101, 10-0201, 10-0301). Les oligonucléotides ont été purifiés par HPLC. Leur pureté a été également contrôlée par analyse HPLC, leur identité a été contrôlée par spectrométrie de masses Maldi-TOF.

Les marqueurs utilisés dans les sondes selon l'invention et les molecular beacons sont :
- l'extincteur de fluorescence Dabsyl (Glen Research, n° cat. 20-5912), noté Da.
- le fluorophore FAM (fluorescéine-amine), Molecular Probes , n° cat. C-2210
- le fluorophore FAM (fluorescéine-amine) attaché à un nucléotide désoxythymine (Glen Research, n° cat. 10-1056), noté dT-FAM.

### Exemple 1 : Etude de l'hybridation des sondes selon l'invention de type alpha des modèles influenza B et HIV avec leur séquence cible.

### Obiectifs :

L'objectif de cette expérience est de démontrer qu'une sonde selon l'invention est capable de produire un signal de fluorescence par hybridation en présence d'une séquence cible complémentaire.

### Design expérimental :

Les sondes utilisées sont les suivantes :
Modèle Influenza B - sonde Opa6d (SEQ ID N°1) :
   5'- gaccgtctg(dT-FAM)GGAGAAGACGTCCAAAAACTGGCAGAC-3'Da Modèle HIV - sonde HOa8 (SEQ ID N°2) :
   5'- accctatct(dT-FAM)CCATCAATGAGGAAGCTGCAGAATGGGATAGAG-3' Da

Les séquences nucléotidiques soulignées correspondent aux séquences de fermeture des sondes selon l'invention.

Les nucléotides écrits en minuscule dans les sondes correspondent à des nucléotides de type *alpha.*

Les séquences cible synthétiques utilisées sont les suivantes :
Modèle Influenza B, cible cC (SEQ ID N° 3) :
   5'- TTTAGTTTTTGGACGTCTTCTCCTTT -3'
Modèle HIV, cible cH4 (SEQ ID N° 4) :
   5'- TTTACTCTATCCCATTCTGCAGCTTCCTCATTGATTTT -3'

Les sondes sont introduites dans une solution contenant :
- un diluant basic kit (bioMérieux B.V., Boxtel, Pays Bas, ref. 70352),
- une sonde à concentration 0,1 micromolaire,
- une séquence cible synthétique à concentration 1,25 micromolaire.

Le volume final est de 20 µl.

Des contrôles négatifs sont préparés en utilisant de l'eau ultrapure MilliQ au lieu de la séquence cible. Cela permet d'observer le comportement de la sonde seule.

Les solutions sont chauffées à 65°C dans le lecteur de fluorescence (EasyQ reader, bioMérieux B.V., Boxtel, Pays Bas) et laissées refroidir pendant 5 heures. La fluorescence de chaque solution est mesurée à 520 nm tout au long du refroidissement.

### Résultats :

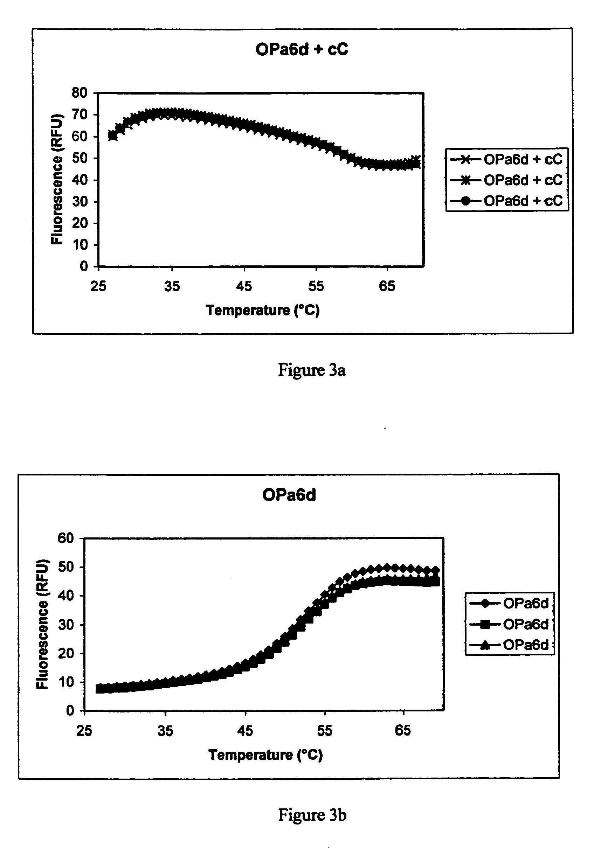

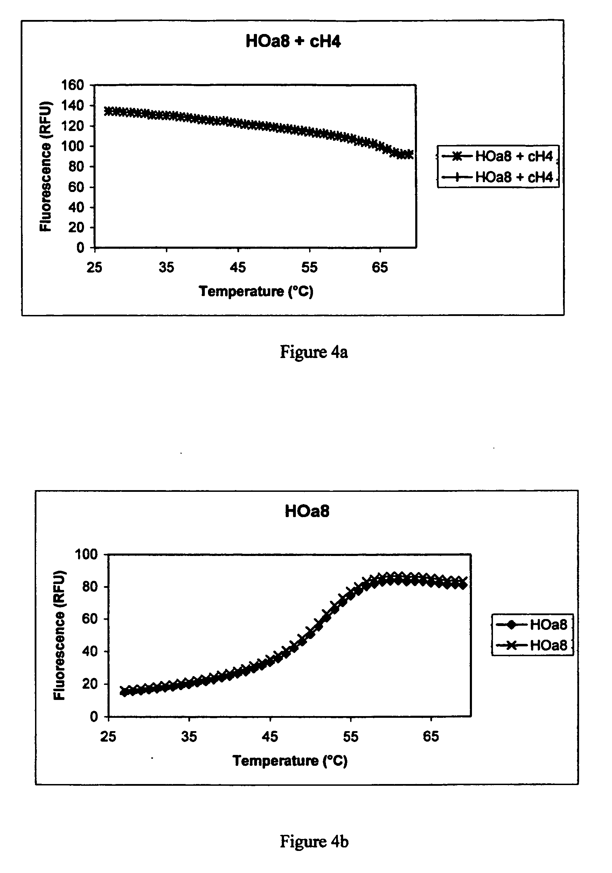

Les résultats obtenus sont représentés dans les figures 3 et 4.

Les sondes Opa6 et Hoa8 en présence de leur séquence cible synthétique respective (figure 3a et 4a) présentent le profil de fluorescence attendu : à basse température, la sonde est hybridée avec sa cible et le fluorophore est à une distance maximale de l'extincteur. La fluorescence est maximale. A haute température, la double hélice sonde/cible est déstabilisée, la sonde devient simple brin et la distance entre le fluorophore et l'extincteur diminue, provocant une diminution de la fluorescence. La stabilité thermique de la double hélice sonde/cible est donnée par la valeur de « Tm-cible ». La Tm correspond au point d'inflexion de la courbe fluorescence = f(température). La Tm-cible est calculée comme le minimum de la première dérivée de la courbe. Les sondes Opa6 et Hoa8 ont une Tm-cible (voir tableau 1) qui leur permet de rester hybridées avec leur cible à la température de la réaction d'amplification NASBA, 41°C, et de produire ainsi un maximum de signal de fluorescence en présence de sa séquence cible.

Les sondes Opa6d et Hoa8 seules (figure 3b et 4b) présentent le profil de fluorescence attendu : à basse température, la sonde est structurée en forme circulaire et le fluorophore est très près de l'extincteur. La fluorescence émise est très faible. A haute température, la structure secondaire est dissociée, le fluorophore s'éloigne de l'extincteur et l'émission de la fluorescence augmente au fur et à mesure et atteint un plateau lorsque la structure secondaire est complètement dénaturée. La stabilité thermique de la structure secondaire de la sonde est donnée par la valeur de « Tm-sonde », qui correspond au point d'inflexion de la courbe fluorescence=f(température). La Tm-sonde est calculée comme le maximum de la première dérivée de la courbe. Les sondes Opa6d et Hoa8 ont une Tm-sonde (voir tableau 1) qui leur permet de rester fermées à la température de la réaction d'amplification NASBA, 41°C, et de produire ainsi un minimum de fluorescence résiduelle en l'absence de sa séquence cible.

Le « ratio ouvert/fermé », aussi appelé « ratio-O/F », permet de mesurer le niveau de signal produit par une sonde en présence de sa cible à 41°C. Il est calculé comme le ratio entre le signal produit par la sonde en présence de sa séquence cible et le signal résiduel produit par la sonde seule.

Dans le tableau 1 sont réunies les valeurs de Tm-sonde, Tm-cible et ratio-O/F obtenus avec les sondes décrites dans cet exemple.

**Tableau 1 : Valeurs de Tm-sonde, Tm-cible et ratio-O/F**

| **Sonde** | **Tm-sonde (°C)** | **Tm-cible (°C)** | **ratio-O/F** |
|---|---|---|---|
| **OPa6d** (SEQ ID N° 1) | 51,8 | 57,7 | 5,4 |
| **HOa8** (SEQ ID N°2) | 51,0 | 64,5 | 4,5 |

### Conclusions :

Les sondes selon l'invention de type *alpha,* des modèles influenza B et HIV, seules en solution, forment une structure secondaire stable à 41°C, produisant une extinction du signal de fluorescence due à la proximité du fluorophore et de l'extincteur.

Lorsqu'elles sont en solution en présence de leur séquence cible synthétique, elles s'hybrident avec leurs séquences cibles synthétiques produisant un signal de fluorescence mesurable.

### Exemple 2 : Etude de l'hybridation des sondes selon l'invention de type inversé des modèles influenza B et HIV avec leur séquence cible.

### Obiectifs :

L'objectif de cette expérience est de démontrer qu'une sonde modifiée selon l'invention est capable de produire un signal de fluorescence par hybridation en présence d'une séquence cible complémentaire.

### Design expérimental :

Les sondes utilisées sont les suivantes :
Modèle Influenza B - sonde Opi6d (SEQ ID N° 5) :
   **5'-Da -GGAGAAGACGTCCAAAAACTGGCAGA-3'-(dT-FAM)-3'-cctcttctg-**5'
Modèle HIV - sonde HOi8d (SEQ ID N°6) :
   5'-Da -CCATCAATGAGGAAGCTGCAGAATGGGATAGAG-3'-(dT-FAM)-3'-**ggtagttactc**-5'

Les séquences soulignées correspondent aux séquences de fermeture des sondes selon l'invention.

Les nucléotides écrits en minuscule et en gras dans les sondes correspondent à des nucléotides inversés.

Les séquences cible synthétiques utilisées sont les suivantes :
Modèle Influenza B, cible cC (SEQ ID N°3) :
   5'- TTTAGTTTTTGGACGTCTTCTCCTTT -3'
Modèle HIV, cible cH4 (SEQ ID N°4) :
   5'- TTTACTCTATCCCATTCTGCAGCTTCCTCATTGATTTT -3'

Les sondes sont introduites dans une solution contenant :
- un diluant basic kit (bioMérieux B.V., Boxtel, Pays Bas, ref. 70352),
- une sonde à concentration 0,1 micromolaire,
- une séquence cible synthétique à concentration 1,25 micromolaire.

Le volume final est de 20 µl.

Des contrôles négatifs sont préparés en utilisant de l'eau ultrapure MilliQ au lieu de la séquence cible. Cela permet d'observer le comportement de la sonde seule.

Les solutions sont chauffées à 65°C dans le lecteur de fluorescence (EasyQ reader, bioMérieux B.V., Boxtel, Pays Bas) et laissées refroidir pendant 5 heures. La fluorescence de chaque solution est mesurée à 520 nm tout au long du refroidissement. Les résultats sont exprimés en forme de graphique de température versus fluorescence.

### Résultats :

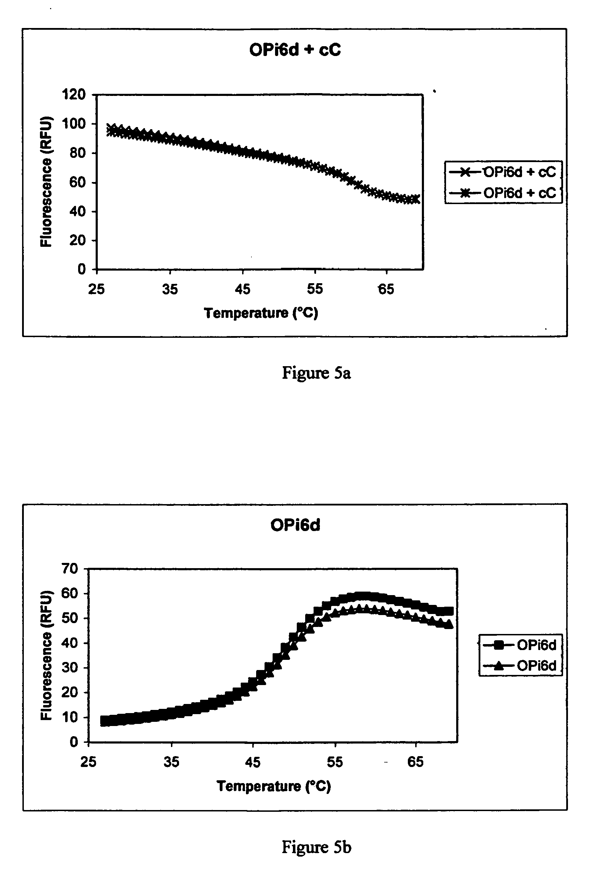

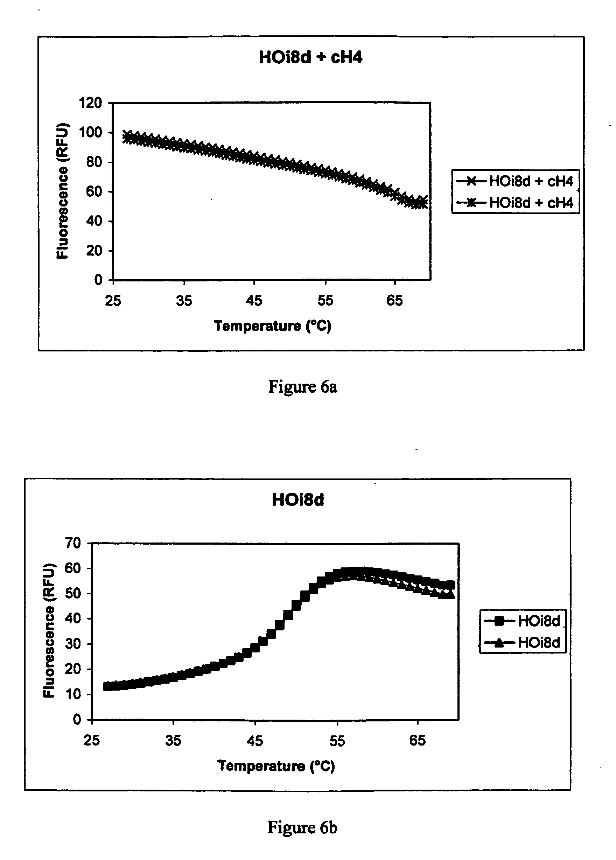

Les résultats obtenus sont représentés dans les figure 5 et 6.

Les sondes Opi6 et Hoi8d en présence de leur séquence cible synthétique respective (figure 5a et 6a) présentent le profil de fluorescence attendu : à basse température, la sonde est hybridée avec sa cible formant un duplex suffisamment rigide pour écarter le fluorophore de l'extincteur. Dans ce duplex, les deux molécules se trouvent à une distance maximale l'une de l'autre. La fluorescence émise est maximale.

A haute température, la double hélice sonde/cible est dissociée, la sonde devient alors simple brin. Dans ce cas, même si la structure secondaire au sein de cette sonde est dissociée, la distance entre le fluorophore et l'extincteur est inférieure à celle dans le duplex sonde/cible à cause de la flexibilité de la sonde dénaturée. Ceci induit une diminution de la fluorescence par extinction. La stabilité thermique de la double hélice sonde/cible est donnée par la valeur de « Tm-cible » qui correspond au point d'inflexion de la courbe fluorescence = f(température). La Tm-cible est calculée comme le minimum de la première dérivée de la courbe. Les sonde Opi6 et Hoi8d ont une Tm-cible (voir tableau 2) qui leur permet de rester hybridées avec leur cible à la température de la réaction d'amplification NASBA, 41°C, et de produire ainsi un maximum de signal de fluorescence en présence de leur séquence cible.

Les sondes Opi6d et Hoi8d seules (figure 5b et 6b) présentent le profil de fluorescence attendu : à basse température, la sonde est structurée en forme circulaire et le fluorophore est très près de l'extincteur. La fluorescence émise est très faible. A haute température, la structure secondaire est dissociée, le fluorophore s'éloigne de l'extincteur et l'émission de la fluorescence augmente au fur et à mesure et atteint un plateau lorsque la structure secondaire est complètement dénaturée. La stabilité thermique de la structure secondaire de la sonde est donnée par la valeur de « Tm-sonde », qui correspond au point d'inflexion de la courbe. La Tm-sonde est calculée comme le maximum de la première dérivée de la courbe fluorescence = f(température). Les sondes Opi6d et Hoi8d ont une Tm-sonde (voir tableau 2) qui leur permet de rester fermées à la température de la réaction d'amplification NASBA, 41°C, et de produire ainsi un minimum de fluorescence résiduelle en l'absence de sa séquence cible.

Le « ratio ouvert/fermé », aussi appelé « ratio-O/F », permet de mesurer le niveau de signal produit par une sonde en présence de sa cible à 41°C. Il est calculé comme le ratio entre le signal produit par la sonde en présence de sa séquence cible et le signal résiduel produit par la sonde seule.

Dans le tableau 2 sont réunies toutes les valeurs de Tm-sonde, Tm-cible et ratio-O/F obtenus avec les sondes décrites dans cet exemple.

**Tableau 2 : Valeurs de Tm-sonde, Tm-cible et ratio-O/F**

| **Sonde** | **Tm-sonde (°C)** | **Tm-cible (°C)** | **rado-O/F** |
|---|---|---|---|
| **OPi6d** (SEQ ID N° 5) | 48,5 | 59,7 | 5,3 |
| **HOi8d** (SEQ ID N°6) | 48,6 | 64,0 | 3,7 |

### Conclusions :

Les sondes selon l'invention de type inversé des modèles influenza B et HIV, seules en solution, forment une structure secondaire stable à 41°C, produisant une extinction du signal de fluorescence due à la proximité du fluorophore et de l'extincteur.

Lorsqu'elles sont en solution en présence de leur séquence cible synthétique, elles s'hybrident avec leurs séquences cibles synthétiques produisant un signal de fluorescence mesurable.

### Exemple 3 : Etude de la détection en temps réel pendant l'amplification NASBA de la cible influenza B par les sondes selon l'invention de type apha.

### Obiectifs :

L'objectif de cette expérience est de démontrer qu'une sonde selon l'invention est capable de détecter sa séquence cible en temps réel pendant l'amplification NASBA.

### Design expérimental :

La séquence cible de l'amplification est un ARN d'une longueur de 212 nucléotides, synthétisé par transcription à partir d'un plasmide selon une technique de transcription *in vitro* connue de l'homme du métier. La séquence de cet ARN correspond à une séquence du génome du virus de l'influenza B.

Les amorces et sondes utilisées sont les suivantes :
Amorces :
   P1 (SEQ ID N°7) :
      5'- *AATTCTAATACGACTCACTATAGGG*GAGCTCTGCTTTAGCACTTCCA -3'
   P2 (SEQ ID N°8) :
      5'- GAGAAATGCAGATGGTCTCAGCTA -3'
Sonde Opa6d (SEQ ID N°1) :
   5'- gaccgtctg(dT-FAM)GGAGAAGACGTCCAAAAACTGGCAGAC-3' Da

Le segment en italique du primer P1 correspond à la séquence du promoteur reconnu par l'enzyme T7 ARN polymérase, nécessaire pour l'amplification enzymatique *in vitro* NASBA.

Les segments soulignés correspondent aux séquences de fermeture des sondes selon l'invention.

Les nucléotides écrits en minuscule dans la sonde Opa6d correspondent à des nucléotides de type *alpha.*

Le segment en 3' de l'amorce P1 est complémentaire d'une séquence de l'ARN cible. La séquence de l'amorce P2 est égale à une séquence de l'ARN cible située du côté 5' de la séquence complémentaire de l'amorce P1. La réaction d'amplification NASBA va générer une multitude de copies d'une séquence ARN, dite « amplicon », dont la séquence correspond à la séquence complémentaire de l'ARN cible, dans le segment compris entre la séquence d'hybridation de l'amorce P1 et la séquence égale à la séquence de l'amorce P2. Les sondes selon l'invention comportent une séquence de reconnaissance qui va s'hybrider avec les amplicons générés par la réaction d'amplification NASBA. De ce fait, le signal produit par la sonde est détecté seulement quand la séquence d'ARN cible est présente dans le mélange réactionnel et qu'elle est amplifiée par la NASBA avec les amorces choisies.

La réaction d'amplification NASBA est réalisée à l'aide des réactifs « NASBA basic kit » (bioMérieux B.V., Boxtel, Pays Bas, ref. 60079136 & 60085192) et dans les conditions prescrites par le fabricant. Chaque tube d'amplification contient un volume final de 20 µl, qui est composé comme suit :
- 8,64 µl des réactifs « NASBA basic kit » dans les proportions prescrites par le fabricant,
- 0,4 µl d'amorce P1 à 10 µM,
- 0,4 µl d'amorce P2 à 10 µM,
- 0,16 µl de la sonde Opa6d à 10 µM,
- 5 µl de la cible ARN à une concentration suffisante pour obtenir une concentration finale de 5, 50, 500 ou 5000 copies par tube réactionnel.

Dans tous les cas, un témoin négatif est réalisé qui contient de l'eau « DNase/RNase free » (n° catalogue 32739, ACROS). Toutes les expériences sont réalisées en double.

Après une étape de dénaturation de 5 minutes à 65°C puis une incubation de 2 minutes à 41°C, 5 µl du reactif « enzyme mix » (1 accusphère « enzyme » 60085111 pour 45 µl de diluant « enzyme » 60085192, partie du « NASBA basic kit ») est ajouté au mélange réactionnel. Le mélange réactionnel est alors introduit dans le lecteur de fluorescence dont la température a été fixée à 41 °C et la réaction est suivie grâce au logiciel Director 2.0 du lecteur de fluorescence (EasyQ reader, bioMérieux B.V, Boxtel, Pays Bas), avec lecture de fluorescence émise par le fluorophore FAM pendant 90 minutes à 520 nm.

A la fin de la réaction, un profil graphique de détection est obtenu pour chaque tube, avec les valeurs numériques de « Alpha3 » et « MSR ». La valeur « Alpha3 » est « le délai d'épuisement des amorces » tel que décrit dans l'article de Weusten et al. (Nucleic Acids Research 2002, vol. 30, N°6, e26). Cette valeur correspond au temps auquel l'augmentation du signal de fluorescence commence à être détectable. Plus l'amplification est rapide et donc efficace, plus la valeur Alpha3 est courte.

La valeur « MSR » est le "Ratio maximum du signal". Cette valeur correspond au ratio entre la valeur de fluorescence lue à la fin de la réaction d'amplification et la valeur de fluorescence finale théorique extrapolée sur les valeurs initiales, avant le début de l'augmentation du signal. Cette valeur est le niveau de signal procuré par la sonde, et doit être supérieur à 1 pour que le signal puisse être considéré positif. Ces deux valeurs sont calculées et affichées automatiquement par le logiciel du lecteur EasyQ pour chaque échantillon.

### Résultats :

Les résultats de détection en NASBA en temps réel ne sont pas montrés mais sont disponibles si nécessaire. On peut observer que la limite de détection se situe à 5 copies de transcript cible. Les échantillons positifs produisent un signal détectable avec un « MSR » optimum de 4,47 et une valeur de « Alpha3 » entre 23 et 31 minutes.

### Conclusions :

La sonde Opa6d selon l'invention est capable d'émettre un signal de fluorescence détectable en présence de sa cible en solution, caractérisé par un niveau -optimum de signal de 4,47 (« MSR »). La valeur de « Alpha3 », qui marque le temps auquel le signal devient détectable, se situe entre 23 et 31 minutes. L'« Alpha3 » est proportionnel à la quantité de cible présente, ce qui donne la possibilité de quantifier la cible à partir de cette valeur.

### Exemple 4 : Etude de la détection en temps réel pendant l'amplification NASBA de la cible influenza B par les sondes selon l'invention de type inversé.

### Obiectifs :

L'objectif de cette expérience est de démontrer qu'une sonde selon l'invention est capable de détecter sa séquence cible en temps réel pendant l'amplification NASBA.

### Design expérimental

La séquence cible de l'amplification est un ARN d'une longueur de 212 nucléotides, synthétisé par transcription à partir d'un plasmide selon une technique de transcription *in vitro* connue de l'homme du métier. La séquence de cet ARN correspond à une séquence du génome du virus de l'influenza B.

Les amorces et sondes utilisées sont les suivantes :
Amorces :
   P1 (SEQ ID N°7) :
      5'- *AATTCTAATACGACTCACTATAGG*GGAGCTCTGCTTTAGCACTTCCA -3'
   P2 (SEQ ID N°8) :
      5'- GAGAAATGCAGATGGTCTCAGCTA -3'
Sonde Opi6d (SEQ ID N°5) :
   5'-Da - GGAGAAGACGTCCAAAAACTGGCAGA-(dT-FAM)- 3'-3'-**cctcttctg**-5'

Les segments soulignés correspondent aux séquences de fermeture des sondes selon l'invention.

Le segment en italique du primer P1 -correspond à la séquence du promoteur reconnu par l'enzyme T7 ARN, polymérase, nécessaire pour l'amplification enzymatique *in vitro* NASBA. Les nucléotides écrits en minuscule et en gras dans la sonde Opi6d correspondent à des nucléotides inversés.

Le segment en 3' de l'amorce P1 est complémentaire d'une séquence de l'ARN cible. La séquence de l'amorce P2 est égale à une séquence de l'ARN cible située du côté 5' de la séquence complémentaire de l'amorce P1. La réaction d'amplification NASBA va générer une multitude de copies d'une séquence ARN, dite « amplicon », dont la séquence correspond à la séquence complémentaire de l'ARN cible, dans le segment compris entre la séquence d'hybridation de l'amorce P1 et la séquence égale à la séquence de l'amorce P2. Les sondes selon l'invention comportent une séquence de reconnaissance qui va s'hybrider avec les amplicons générés par la réaction d'amplification NASBA. De ce fait, le signal produit par la sonde est détecté seulement quand la séquence d'ARN cible est présente dans le mélange réactionnel et qu'elle est amplifiée par la NASBA avec les amorces choisies.

La réaction d'amplification NASBA est réalisée à l'aide des réactifs « NASBA basic kit » (bioMérieux B.V., Boxtel, Pays Bas, réf. : 60079136 et 60085192) et dans les conditions prescrites par le fabricant. Chaque tube d'amplification contient un volume final de 20 µl, qui est composé comme suit :
- 8,64 µl des réactifs « NASBA basic kit » dans les proportions prescrites par le fabricant,
- 0,4 µl d'amorce P1 à 10 µM,
- 0,4 µl d'amorce P2 à 10 µM,
- 0,16 µl de la sonde Opi6d à 10 µM,
- 5µl de la cible ARN à une concentration suffisante pour obtenir une concentration finale de 5, 50, 500 ou 5000 copies par tube réactionnel.

Dans tous les cas, un témoin négatif est réalisé qui contient de l'eau « DNase/RNase free » (n° catalogue 32739, ACROS).

Après une étape de dénaturation de 5 minutes à 65°C puis une incubation de 2 minutes à 41°C, 5µl du reactif « enzyme mix » (1 accusphère « enzyme » ref. 60085111 pour 45µl de diluant « enzyme » ref. 60085192, partie du « NASBA basic kit ») est ajouté au mélange réactionnel. Le mélange réactionnel est alors introduit dans le lecteur de fluorescence dont la température a été fixée à 41°C et la réaction est suivie avec le logiciel Director 2.0 du lecteur de fluorescence (EasyQ reader, bioMérieux B.V., Boxtel, Pays Bas), avec lecture de fluorescence du fluorophore FAM pendant 90 minutes à 520 nm.

A la fin de la réaction, un profil graphique de détection est obtenu pour chaque tube, ensemble avec les valeurs numériques de « Alpha3 » et « MSR ».

### Résultats :

Les résultats de détection en NASBA en temps réel ne sont pas montrés mais sont disponibles si nécessaires. On peut observer que la limite de détection se situe à 5 copies de transcript cible. Les échantillons positifs produisent un signal détectable avec un « MSR » optimum de 3,88 et une valeur de « Alpha3 » entre 18 et 26 minutes.

### Conclusions :

La sonde Opi6d selon l'invention est capable d'émettre un signal de fluorescence détectable en présence de sa cible en solution, caractérisé par un niveau optimum de signal de 3,88 (« MSR »). La valeur de « Alpha3 », qui marque le temps auquel le signal devient détectable, se situe entre 18 et 26 minutes. L' « Alpha3 » est proportionnel à la quantité de cible présente, ce qui donne la possibilité de quantifier la cible à partir de cette valeur.

### Exemple 5 : Etude de la détection en temps réel pendant l'amplification NASBA de la cible HIV par les sondes selon l'invention de type alpha,

### Obiectifs :

L'objectif de cette expérience est de démontrer qu'une sonde selon l'invention est capable de détecter sa séquence cible en temps réel pendant l'amplification NASBA.

### Design expérimental :

La séquence cible de l'amplification est un ARN d'une longueur de 212 nucléotides, synthétisé par transcription à partir d'un plasmide selon une technique de transcription *in vitro* connue de l'homme du métier. La séquence de cet ARN correspond à une séquence du génome du virus de l'immunodéficience humaine « HIV ».

Les amorces et sondes utilisées sont les suivantes :
Amorces :
   P3 (SEQ ID N°9) :
      5'- *AATTCTAATACGACTCACTATAGGG*TGCTATGTCACTTCCCCTTGGTTCTCTCA -3' P4 (SEQ ID N°10) :
      5'- AGTGGGGGGACATCAAGCAGCCATGCAAA -3'
Sonde Hoa6 (SEQ ID N°11) :
   5'- accctatcc-(dT-FAM)-ATCAATGAGGAAGCTGCAGAATGGGATAGG -3'-Da

Les segments soulignés correspondent aux séquences de fermeture des sondes selon l'invention.

Le segment en italique du primer P3 correspond à la séquence du promoteur reconnue par l'enzyme T7 ARN polymérase, nécessaire pour l'amplification enzymatique *in vitro* NASBA. Les nucléotides écrits en minuscule dans la sonde Hoa6 correspondent à des nucléotides *alpha.*

Le segment en 3' de l'amorce P3 est complémentaire d'une séquence de l'ARN cible. La séquence de l'amorce P4 est égale à une séquence de l'ARN cible située du côté 5' de la séquence complémentaire de l'amorce P3. La réaction d'amplification NASBA va générer une multitude de copies d'une séquence ARN, dite « amplicon », dont la séquence correspond à la séquence complémentaire de l'ARN cible, dans le segment compris entre la séquence d'hybridation de l'amorce P3 et la séquence égale à la séquence de l'amorce P4. Les sondes selon l'invention comportent une séquence de reconnaissance qui va s'hybrider avec les amplicons générés par la réaction d'amplification NASBA. De ce fait, le signal produit par la sonde est détecté seulement quand la séquence d'ARN cible est présente dans le mélange réactionnel et qu'elle est amplifiée par la NASBA avec les amorces choisies.

La réaction d'amplification NASBA est réalisée à l'aide des réactifs « NASBA basic kit » (bioMérieux B.V., Boxtel, Pays Bas, ref. 60079136 et 60085192) et dans les conditions prescrites par le fabricant. Chaque tube d'amplification contient un volume final de 20 µl, qui est composé comme suit :
- 8,64 µl des réactifs « NASBA basic kit » dans les proportions prescrites par le fabricant,
- 0,4 µl d'amorce P3 à 10 µM,
- 0,4 µl d'amorce P4 à 10 µM,
- 0,16 µl de la sonde Hoa6 à 10 µM,
- 5µl de la cible ARN à une concentration suffisante pour obtenir une concentration finale de 5, 50, 500 ou 5000 copies par tube réactionnel.

Dans tous les cas, un témoin négatif est réalisé qui contient de l'eau « DNase/RNase free » (n° catalogue 32739, ACROS).

Après une étape de dénaturation de 5 minutes à 65°C puis une incubation de 2 minutes à 41°C, 5 µl du réactif « enzyme mix » (1 accusphère « enzyme » réf. 60085111 pour 45 µl de diluant « enzyme » réf. 60085192, partie du « NASBA basic kit ») est ajouté au mélange réactionnel. Le mélange réactionnel est alors introduit dans le lecteur de fluorescence dont la température a été fixée à 41°C et la réaction est suivie avec le logiciel Director 2.0 du lecteur de fluorescence (EasyQ reader, bioMérieux B.V., Boxtel, Pays Bas), avec lecture de fluorescence du fluorophore FAM pendant 90 minutes à 520 nm.

A la fin de la réaction, un profil graphique de détection est obtenu pour chaque tube, ensemble avec les valeurs numériques de « Alpha3 » et « MSR ».

### Résultats :

Les résultats de détection en NASBA en temps réel ne sont pas montrés mais sont disponibles si nécessaire. On peut observer que la limite de détection est égale ou inférieure à 5 copies de transcript cible. Les échantillons positifs produisent un signal détectable avec un « MSR » optimum de 2,53 et une valeur de « Alpha3 » entre 14 et 22 minutes.

### Conclusions :

La sonde Hoa6 selon l'invention est capable d'émettre un signal de fluorescence détectable en présence de sa cible en solution, caractérisé par un niveau optimum de signal de 2,53 (« MSR »). La valeur de « Alpha3 », qui marque le temps auquel le signal devient détectable, se situe entre 14 et 22 minutes. L' « Alpha3 » est proportionnel à la quantité de cible présente, ce qui donne la possibilité de quantifier la cible à partir de cette valeur.

### Exemple 6 : Etude de la détection en temps réel pendant l'amplification NASBA de la cible HIV par les sondes selon l'invention de type inversé.

### Obiectifs :

L'objectif de cette expérience est de démontrer qu'une sonde selon l'invention est capable de détecter sa séquence cible en temps réel pendant l'amplification NASBA.

### Design expérimental :

La séquence cible de l'amplification est un ARN d'une longueur de 212 nucléotides, synthétisé par transcription à partir d'un plasmide selon une technique de transcription *in vitro* connue de l'homme du métier. La séquence de cet ARN correspond à une séquence du génome du virus de l'immunodéficience humaine « HIV ».

Les amorces et sondes utilisées sont les suivantes :
Amorces :
   P3 (SEQ ID N°9) :
      5'- *AATTCTAATACGACTCACTATAGGG*TGCTATGTCACTTCCCCTTGGTTCTCTCA -3'
   P4 (SEQ ID N°10) :
      5'- AGTGGGGGGACATCAAGCAGCCATGCAAA -3'
Sonde Hoi6d (SEQ ID N°12) :
   5'-Da- CCATCAATGAGGAAGCTGCAGAATGGGATAGG-3'-(dT-FAM) 3'-**ggtagttact** -5'

Les segments soulignés correspondent aux séquences de fermeture des sondes selon l'invention.

Les nucléotides écrits en minuscule et en gras dans les sondes correspondent à des nucléotides inversés.

Le segment en italique du primer P3 correspond à la séquence du promoteur reconnu par l'enzyme T7 ARN polymérase, nécessaire pour l'amplification enzymatique *in vitro* NASBA. Les nucléotides écrits en minuscule et en gras dans la sonde Hoi6d correspondent à des nucléotides inversés.

Le segment en 3' de l'amorce P3 est complémentaire d'une séquence de l'ARN cible. La séquence de l'amorce P4 est égale à une séquence de l'ARN cible située du côté 5' de la séquence complémentaire de l'amorce P3. La réaction d'amplification NASBA va générer une multitude de copies d'une séquence ARN, dite « amplicon », dont la séquence correspond à la séquence complémentaire de l'ARN cible, dans le segment compris entre la séquence d'hybridation de l'amorce P3 et la séquence égale à la séquence de l'amorce P4. Les sondes selon l'invention comportent une séquence de reconnaissance qui va s'hybrider avec les amplicons générés par la réaction d'amplification NASBA. De ce fait, le signal produit par la sonde est détecté seulement quand la séquence d'ARN cible est présente dans le mélange réactionnel et qu'elle est amplifiée par la NASBA avec les amorces choisies.

La réaction d'amplification NASBA est réalisée à l'aide des réactifs « NASBA basic kit » (bioMérieux B.V., Boxtel, Pays Bas, ref. 60079136 et 60085192) et dans les conditions prescrites par le fabricant. Chaque tube d'amplification contient un volume final de 20 µl, qui est composé comme suit :
- 8,64 µl des réactifs « NASBA basic kit » dans les proportions prescrites par le fabricant,
- 0,4 µl d'amorce P3 à 10 µM,
- 0,4 µl d'amorce P4 à 10 µM,
- 0,16 µl de la sonde Hoi6d à 10 µM,
- 5µl de la cible ARN à une concentration suffisante pour obtenir une concentration finale de 5, 50, 500 ou 5000 copies par tube réactionnel.

Dans tous les cas, un témoin négatif est réalisé qui contient de l'eau « DNase/RNase free » (n° catalogue 32739, ACROS).

Après une étape de dénaturation de 5 minutes à 65°C puis une incubation de 2 minutes à 41°C, 5 µl du reactif « enzyme mix » (1 accusphère « enzyme » ref. 60085111 pour 45µl de diluant « enzyme » ref. 60085192, partie du « NASBA basic kit ») est ajouté au mélange réactionnel. Le mélange réactionnel est alors introduit dans le lecteur de fluorescence dont la température a été fixée à 41°C et la réaction est suivie avec le logiciel Director 2.0 du lecteur de fluorescence (EasyQ reader, bioMérieux B.V., Boxtel, Pays Bas), avec lecture de fluorescence du fluorophore FAM pendant 90 minutes à 520 nm.

A la fin de la réaction, un profil graphique de détection est obtenu pour chaque tube avec les valeurs numériques de « Alpha3 » et « MSR ».

### Résultats :

Les résultats de détection en NASBA en temps réel ne sont pas montrés mais sont disponibles si nécessaire. On peut observer que la limite de détection est égale ou inférieure à 5 copies de transcript cible. Les échantillons positifs produisent un signal détectable avec un « MSR » optimum de 2,03 et une valeur de « Alpha3 » entre 15 et 21 minutes.

### Conclusions :

La sonde Hoi6d selon l'invention est capable d'émettre un signal de fluorescence détectable en présence de sa cible en solution, caractérisé par un niveau optimum de signal de 2,03 (« MSR »). La valeur de « Alpha3 », qui marque le temps auquel le signal devient détectable, se situe entre 15 et 21 minutes. L'« Alpha3 » est proportionnel à la quantité de cible présente, ce qui donne la possibilité de quantifier la cible à partir de cette valeur.

### Exemple 7 : Etude de la spécificité des sondes selon l'invention sur le modèle Influenza B.

### Obiectifs :

La spécificité d'une sonde est sa capacité à reconnaître uniquement sa cible, et ainsi, de la différencier d'une cible comportant par exemple des mésappariements ou délétions. Les molecular beacons sont aujourd'hui les sondes moléculaires les plus spécifiques puisqu'ils permettent la différenciation de deux cibles possédant seulement un nucléotide différent. L'objectif de cette expérience est de montrer le niveau de spécificité des sondes selon l'invention, en comparaison avec la sonde molecular beacon qui contient la même séquence de reconnaissance.

### Design expérimental :

Les sondes utilisées sont les suivantes :
Molecular beacon CP InfB (SEQ ID N°13) :
   5'- (FAM)-CGATCGGGAGAAGACGTCCAAAAACTCGATCG -3'Da
Sonde Opa6 (SEQ ID N°14) :
   5'- gagcgtagc(dT-FAM)GGAGAAGACGTCCAAAAACTCGCATCG -3' Da
Sonde Opa5 (SEQ ID N°15) :
   5'- gagctagc(dT-FAM)GGAGAAGACGTCCAAAAACTCGATCG-3' Da
Sonde Opi4 (SEQ ID N°16) :
   3'- **gagctagc**-5'-5'-(dT-FAM)GGAGAAGACGTCCAAAAACTCGATCG -3' Da
Sonde Opi3 (SEQ ID N°17) :
   3'- **gctagcc**-5'-5'-(dT-FAM)GGAGAAGACGTCCAAAAACTCGATCGG -3' Da

Les segments soulignés correspondent aux séquences de fermeture des sondes selon l'invention et aux séquences des brins formant la partie « tige » du molecular beacon.

Les nucléotides écrits en minuscule dans les sondes correspondent à des nucléotides de type *alpha.*

Les nucléotides écrits en minuscule et en gras dans les sondes correspondent à des nucléotides inversés.

Les séquences cibles synthétiques utilisées sont les suivantes :
Cible cC (SEQ ID N°3) :
   5'- TTTAGTTTTTGGACGTCTTCTCCTTT -3'
Cible cG (SEQ ID N°18) :
   5'- TTTAGTTTTTGGAGGTCTTCTCCTTT -3'
Cible cA (SEQ ID N°19) :
   5'- TTTAGTTTTTGGAAGTCTTCTCCTTT -3'
Cible cT (SEQ ID N°20) :
   5'- TTTAGTTTTTGGATGTCTTCTCCTTT -3'

La séquence cible synthétique « cC » est parfaitement complémentaire d'une partie des séquences des sondes. Les séquences cibles synthétiques « cG », « cA » et « cT » contiennent un mésappariement par rapport à la séquence complémentaire dans les sondes, signalé par le soulignage de la lettre correspondante.

Les sondes sont introduites dans une solution contenant :
- Un diluant basic kit (bioMérieux B.V., Boxtel, Pays Bas, ref. 70352 ),
- Une sonde à concentration 0,1 micromolaire,
- Une séquence cible synthétique à concentration 1,25 micromolaire.

Le volume final est de 20 µl.

Des contrôles négatifs sont préparés en utilisant de l'eau ultrapure MilliQ au lieu de la séquence cible. Cela permet d'observer le comportement de la sonde seule.

Les solutions sont chauffées à 65°C dans le lecteur de fluorescence (EasyQ reader, bioMérieux B.V., Boxtel, Pays Bas) et laissées refroidir pendant 5 heures. La fluorescence de chaque solution est mesurée à 520 nm tout au long du refroidissement. Les résultats sont exprimés en forme de graphique de température (en °C) versus fluorescence (en RFU) .

### Résultats :

Parmi les sondes testées pour le modèle Influenza B, OPa6 est celle qui présente le plus grand écart de Tm-cible entre la cible parfaite (cC) et les cibles possédant un mésappariement (cG, cA ou cT).

**Tableau 3 : Tm analytique des sondes du modèle Influenza B (deux premières colonnes de gauche) et écart obtenu entre les Tm cible avec la cible parfaite (cC) et les cibles possédant un mésappariement (colonnes cG et Δ TmcG ; cA Δ Tm cA ; cT et Δ Tm cT).**

| | Tm tige | Tm cible cC (SEQ ID N°3) | Tm cible cG (SEQ ID N°18) | | Tm cible cA (SEQ ID N°19) . | | Tm cible cT (SEQ ID N°20) | |
|---|---|---|---|---|---|---|---|---|
| | | | | Δ Tm | | Δ Tm | | Δ Tm |
| CP InfB (SEQ ID N°13) | 49,7 | 57,7 | 51,0 | -6,7 | 50,2 | -7,5 | 48,2 | -9,5 |
| Opa 6 (SEQ ID N° 14) | 48,8 | 57,5 | 49,8 | -7,6 | 48,5 | -8,9 | 47,1 | -10,4 |
| Opa 5 (SEQ ID N° 15) | 45,1 | 57,6 | 51,8 | -5,8 | 51,2 | -6,4 | 48,9 | -8,8 |
| Opi 4 (SEQ ID N° 16) | 51,8 | 57,1 | 49,7 | -7,3 | 48,9 | -8,1 | 47,0 | -10,1 |
| Opi 3 (SEQ ID N° 17) | 49,5 | 57,5 | 49,8 | -7,7 | 47,7 | -10,0 | 50,6 | -6,9 |

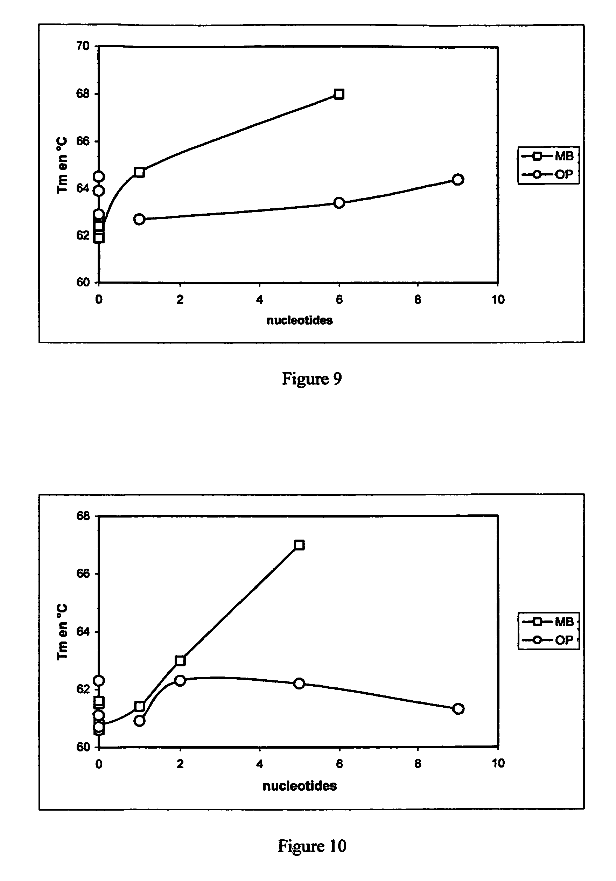

Ce tableau recueille les Tm mesurées à partir des graphiques obtenus. Dans la figure 9 sont représentés deux exemples de ces graphiques. La différence entre la Tm-cible de la sonde avec sa cible parfaite et les Tm-cible de la sonde avec les cibles possédant un mésappariement est bien visible sur les graphiques et peut atteindre 10°C pour les sondes OPi4 et OPa6. La Tm-cible de la sonde avec les cibles possédant un mésappariement étant très proche de la Tm-tige de la sonde, celle-ci se ferme sur elle-même avant de s'hybrider avec sa cible.

### Conclusions :

Les sondes selon l'invention sont des sondes spécifiques d'une cible donnée, leurs performances dans ce domaine sont similaires à celles du molecular beacon de référence. Cela présente un intérêt majeur pour la détection des SNP ou *Single Nucleotide Polymorphismes* correspondant à la variation d'un seul nucléotide dans un gène donné.

### Exemple 8 : Etude de la spécificité des sondes selon l'invention sur le modèle HIV.

### Objectifs :

La spécificité d'une sonde est sa capacité à reconnaître uniquement sa cible, et ainsi, de la différencier d'une cible comportant par exemple des mésappariements ou délétions. Les molecular beacon sont aujourd'hui les sondes moléculaires les plus spécifiques puisqu'ils permettent la différenciation de deux cibles possédant seulement un nucléotide différent. L'objectif de cette expérience est de montrer le niveau de spécificité des sondes selon l'invention, en comparaison avec la sonde molecular beacon qui contient la même séquence de reconnaissance.

### Design expérimental :

Les sondes utilisées sont les suivantes :
Molecular beacon HIV-1A (SEQ ID N°21) :
   5' (FAM)-CTATCCCATCAATGAGGAAGCTGCAGAATGGGATAG-3' Da
Sonde Hoa6 (SEQ ID N°11) :
   5'-accctatcc(dT-FAM)ATCAATGAGGAAGCTGCAGAATGGGATAGG- 3' Da
Hoa7 (SEQ ID N°22) :
   5'-ttaccctatc(dT-FAM)ATCAATGAGGAAGCTGCAGAATGGGATAG- 3' Da
Sonde Hoa8 (SEQ ID N°2) :
   5'-accctatctc(dT-FAM)CCATCAATGAGGAAGCTGCAGAATGGGATAGAG- 3' Da
Sonde Hoi6d (SEQ ID N°12) :
   5'-Da-CCATCAATGAGGAAGCTGCAGAATGGGATAGG-3'-(dT-FAM)-3'-**ggtagttact** -5'
Sonde Hoi7d (SEQ ID N°23) :
   5'-Da-CCATCAATGAGGAAGCTGCAGAATGGGATAGG-3'-(dT-FAM)-3'-**ggtagttactc**-5'
Sonde Hoi8d (SEQ ID N°6) :
   5'-Da-CCATCAATGAGGAAGCTGCAGAATGGGATAGAG-3'-(dT-FAM)-3'-**ggtagttactc**-5'

Les segments soulignés correspondent aux séquences de fermeture des sondes selon l'invention et aux séquences des brins formant la partie « tige » du molecular beacon. Les nucléotides écrits en minuscule dans les sondes selon l'invention correspondent à des nucléotides de type *alpha.*

Les nucléotides écrits en minuscule et en gras dans les sondes selon l'invention correspondent à des nucléotides inversés.

Les séquences cibles synthétiques utilisées sont les suivantes :
Cible cH5 (SEQ ID N° 24) :
   5'- TTTCATTCTGCAGCTTCCTCATTT -3'
Cible cGH5 (SEQ ID N° 25) :
   5'- TTTCATTCTGCAGGTTCCTCATTT -3'
Cible cAH5 (SEQ ID N° 26) :
   5'- TTTCATTCTGCAGATTCCTCATTT -3'
Cible cTH5 (SEQ ID N° 27) :
   5'- TTTCATTCTGCAGTTTCCTCATTT -3'

La séquence cible synthétique « cH5 » est parfaitement complémentaire d'une partie des séquences des sondes. Les séquences cibles synthétiques « cGH5 », « cAH5 » et « cTH5 » contiennent un mésappariement par rapport à la séquence complémentaire dans les sondes, signalé par le soulignage de la lettre correspondante.

Les sondes sont introduites dans une solution contenant :
- Un diluant basic kit (bioMérieux B.V., Boxtel, Pays Bas, ref. 70352).
- Une sonde à concentration 0,1 micromolaire.
- Une séquence cible synthétique à concentration 1,25 micromolaire.

Le volume final est de 20 µl.

Des contrôles négatifs sont préparés en utilisant de l'eau ultrapure MilliQ au lieu de la séquence cible. Cela permet d'observer le comportement de la sonde seule.

Les solutions sont chauffées à 65°C dans le lecteur de fluorescence (EasyQ reader, bioMérieux B.V., Boxtel, Pays Bas) et laissées refroidir pendant 5 heures. La fluorescence de chaque solution est mesurée tout au long du refroidissement à 520 nm. Les résultats sont exprimés en forme de graphique de température versus fluorescence.

### Résultats :

Les résultats sont présentés dans le tableau ci-dessous et dans la figure 10.

**Tableau 4 : Valeurs de Tm-cible du molecular beacon et des sondes selon l'invention de type alpha et inversé avec la cible parfaite cH5 (deux première colonnes de gauche) et écart obtenu entre la Tm-cible des sondes avec la cible parfaite (cH5) et les Tm-cible des sondes avec les cibles possédant un mésappariement (cAH5, cGH5, cTH5) (colonnes Δ Tm cAH5 ; Δ Tm cGH5 ; Δ Tm cTH5).**

| | Tm tige cH5 (SEQ ID N° 24) | Tm cible cH5 (SEQ ID N° 24) | Δ Tm cGH5 (SEQ ID N° 25) | Δ Tm cAH5 (SEQ ID N° 26) | Δ Tm cTH5 (SEQ ID N° 27) |
|---|---|---|---|---|---|
| HIV-1A (SEQ ID N°21) | 50,9 | 58,0 | -6,1 | -7,3 | -8,5 |
| HOa6 (SEQ ID N° 11) | 49,0 | 58,3 | -6,4 | -7,5 | -8,8 |
| HOa7 (SEQ ID N°22) | 44,2 | 58,4 | -6,5 | -7,7 | -8,9 |
| HOa8 (SEQ ID N°2) | 49,9 | 58,4 | -6,5 | -7,7 | -8,9 |
| Hoi6d (SEQ ID N° 12) | 44,1 | 58,5 | -6,7 | -7,8 | -9,1 |
| Hoi7d (SEQ ID N°23) | 48,3 | 58,5 | -6,6 | -7,8 | -9,0 |
| Hoi8d (SEQ ID N°6) | 48,6 | 58,4 | -6,5 | -7,7 | -8,9 |

Ce tableau recueille les Tm mesurées à partir des graphiques obtenus. Dans la figure 10 sont représentés deux exemples de ces graphiques. La différence entre la Tm avec la cible parfaitement complémentaire et les Tm avec les cibles qui contiennent un mésappariement est bien visible et peut atteindre 9°C pour les sondes HOi6d et HOi7d.

### Conclusions :

Les sondes selon l'invention sont des sondes spécifiques d'une cible donnée, leurs performances dans ce domaine dépassent même celles du molecular beacon de référence HIV-1A. Cela présente un intérêt majeur pour la détection des SNP ou *Single Nucleotide Polymorphismes* correspondant à la variation d'un seul nucléotide dans un gène donné.

### Exemple 9 : Etude de l'hybridation spécifique des sondes selon l'invention de type alpha du modèle influenza B avec leur cible.

### Obiectifs :

L'objectif de cette expérience est de démontrer qu'une sonde selon l'invention s'hybride sur un acide nucléique cible d'une manière plus spécifique qu'une sonde molecular beacon.

D'une manière générale, lors du design d'une sonde molecular beacon, la Tm prise en considération est celle calculée à partir de la séquence de reconnaissance, séquence complémentaire de la séquence cible (Tm calculée). En effet, les séquences des brins formant la partie « tige » du molecular beacon sont censées ne pas s'hybrider avec l'acide nucléique cible. Or la Tm réelle de la sonde molecular beacon peut être légèrement supérieure à celle qui est calculée, du fait des interactions des séquences des brins formant la partie « tige » du molecular beacon avec la séquence cible. De plus, les brins formant la partie « tige » peuvent s'hybrider de façon non spécifique avec la séquence de la cible, ce qui a pour effet d'augmenter la stabilité globale de l'hybridation du molecular beacon sur la cible (la Tm-cible réelle est supérieure à la Tm-cible calculée) et de diminuer ainsi la spécificité de cette hybridation.

### Design expérimental :

Les sondes utilisées sont les suivantes :
Sonde Opa6d (SEQ ID N°1) :
   5'- gaccgtctg(dT-FAM)GGAGAAGACGTCCAAAAACTGGCAGAC-3' Da
Molecular beacon CpinfB (SEQ ID N°13) :
   5'- (FAM)-CGATCGGGAGAAGACGTCCAAAAACTCGATCG -3'Da

Les segments soulignés correspondent aux séquences de fermeture des sondes selon l'invention ou aux brins de la partie « tige » du molecular beacon.

Les nucléotides écrits en minuscule dans les sondes correspondent à des nucléotides de type *alpha.*

Les séquences cibles synthétiques utilisées sont les suivantes :
Cible Bioa2I (SEQ ID N°28) :
   5'- TTTAGTTTTTGGACGTCTTCTCCTTTTCCAATT -3'
Cible 6Ba2I (SEQ ID N°29) :
   5'- TTTAGTTTTTGGACGTCTTCTCCCGATCGAATT -3'
Cible 10Pa2I (SEQ ID N°30) :
   5'- TTTAGTTTTTGGACGTCTTCTCCACAGACGGTC -3'
Cible 6Pa2I (SEQ ID N°31) :
   5'- TTTAGTTTTTGGACGTCTTCTCCACAGACAATT -3'
Cible 1Ba2I (SEQ ID N°32) :
   5'- TTTAGTTTTTGGACGTCTTCTCCCTTTCCAATT -3'
Cible 1Pa2I (SEQ ID N°33) :
   5'- TTTAGTTITTGGACGTCTTCTCCATTTCCAATT -3'
Cible 2Ba2I (SEQ ID N°34) :
   5'- TTTAGTTTTTGGACGTCTTCTCCCGTTCCAATT -3'
Cible 2Pa2I (SEQ ID N°35) :
   5'- TTTAGTTTTTGGACGTCTTCTCCACTTCCAATT -3'

La séquence soulignée en pointillé correspond à la partie hybridable avec la séquence de reconnaissance soit du molecular beacon CpinfB (SEQ ID N°13), soit de la sonde selon l'invention Opa6d (SEQ ID N°1).

La séquence Bioa21 (SEQ ID N°28) correspond à un fragment de la séquence cible biologique du virus influenza B.

La séquence cible 6Ba2I (SEQ ID N°29) contient, par rapport à la séquence cible Bioa2I (SEQ ID N°28), six nucléotides en 3' de la séquence soulignée avec des pointillés. Ces six nucléotides, soulignés d'un double trait, sont complémentaires de la séquence de l'un des brins de la partie « tige » du molecular beacon CpinfB (SEQ ID N°13).

La séquence cible 10Pa2I (SEQ ID 30) contient, par rapport à la séquence cible Bioa21 (SEQ ID N°28), dix nucléotides en 3' de la séquence surlignée avec des pointillés. Ces dix nucléotides, soulignés d'un double trait, sont complémentaires d'une des séquences de fermeture de la sonde selon l'invention Opa6d (SEQ ID N°1).

La séquence cible 6Pa2I (SEQ ID N°31) contient, par rapport à la séquence cible Bioa2I (SEQ ID N°28), six nucléotides en 3' de la séquence soulignée avec des pointillés. Ces six nucléotides, soulignés d'un double trait, sont complémentaires d'une des séquences de fermeture de la sonde selon l'invention Opa6d (SEQ ID N°1).

La séquence cible 1Ba2I (SEQ ID N°32) contient, par rapport à la séquence Bioa2I (SEQ ID N°28), un nucléotide en 3' de la séquence soulignée avec des pointillés. Ce nucléotide, souligné d'un double trait, est complémentaire d'un nucléotide de la séquence de l'un des brins de la partie « tige » du molecular beacon CpinfB (SEQ ID N°13).

La séquence cible IPa2I (SEQ ID N°33) contient, par rapport à la séquence Bioa2I (SEQ ID N°28), un nucléotide en 3' de la séquence soulignée avec des pointillés. Ce nucléotide, souligné d'un trait double, est complémentaire d'une des séquences de fermeture de la sonde selon l'invention Opa6d (SEQ ID N°1).

La séquence cible 2Ba2I (SEQ ID N°34) contient, par rapport à la séquence Bioa2I (SEQ ID N°28), deux nucléotides en 3' de la séquence surlignée avec des pointillés. Ces deux nucléotides, soulignés d'un double trait, sont complémentaires de la séquence de l'un des brins de la partie « tige » du molecular beacon CpinfB (SEQ ID N°13).

La séquence cible 2Pa2I (SEQ ID N°35) contient, par rapport à la séquence cible Bioa2I (SEQ ID N°28), deux nucléotides en 3' de la séquence soulignée avec des pointillés. Ces deux nucléotides, soulignés d'un double trait, sont complémentaires d'une des séquences de fermeture sonde selon l'invention Opa6d (SEQ ID N°1).

Les sondes sont introduites dans une solution contenant :
- Diluant basic kit (bioMérieux B.V, Boxtel, Pays Bas, réf. 70352),
- Sonde à concentration 0,1 micromolaire,
- Séquence cible synthétique à concentration 1,25 micromolaire.

Le volume final est de 20 µl.

Des contrôles négatifs sont faits avec de l'eau à la place de la séquence cible. Cela permet d'observer le comportement de la sonde seule.

Les solutions sont chauffées à 68°C dans le lecteur de fluorescence (EasyQ reader, bioMérieux B.V, Boxtel, Pays Bas) et laissées refroidir pendant 5 heures. La fluorescence de chaque solution est mesurée à 520 nm tout au long du refroidissement. La stabilité thermique de la double hélice sonde/cible est donnée par la valeur de « Tm-cible » qui correspond au point d'inflexion de la courbe fluorescence=f(température). La Tm-cible est calculée comme le minimum de la première dérivée de cette courbe.

### Résultats :

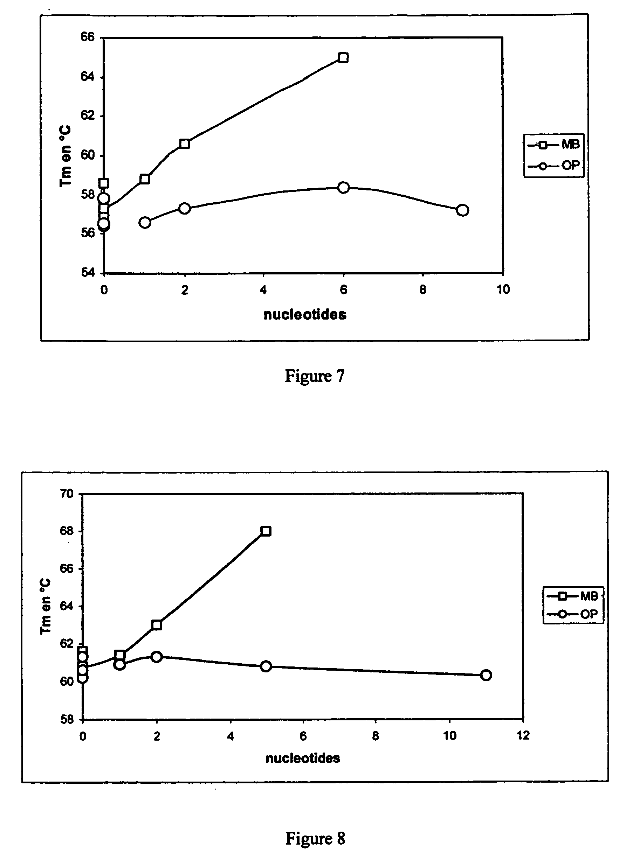

Les résultats sont présentés dans le tableau 5 et sur la figure 7. Le tableau 5 réunit les valeurs de Tm-cible (en °C) obtenues avec les sondes et cibles décrites dans cet exemple.

L'augmentation de la Tm-cible par rapport à une Tm-cible de référence indique la formation d'interactions supplémentaires entre la cible et sa sonde. Dans cet exemple, les Tm-cible de référence correspondent aux Tm-cible mesurées entre le molecular beacon CpinfB3 (SEQ ID N°13) et la cible Bioa2I (SEQ ID N°28) et à celle mesurée entre la sonde selon l'invention Opa6d (SEQ ID N°1) et la cible Bio2aI (SEQ ID N°28). Elles sont respectivement égale à 57,3°C et 56,4°C.

**Tableau 5 : Présentation des valeurs de Tm-cible obtenues avec les différentes sondes.**

| | | **Sondes** | |
|---|---|---|---|
| | | **CPInfB(SEQ ID N°13)** | **Opa6d (SEQ ID N°1)** |
| **Séquences** | **Bioa2I (SEQ ID N°28)** | 57,3 | 56,4 |
| **cible** | **6Ba2I (SEQ ID N°29)** | 65 | 57,8 |
| | **10Pa2I (SEQ ID N°30)** | 57,2 | 57,2 |
| | **6Pa2I (SEQ ID N°31)** | 58,6 | 58,4 |
| | **1Ba2I (SEQ ID N°32)** | 58,8 | 56,5 |
| | **1Pa2I (SEQ ID N°33)** | 57 | 56,6 |
| | **2Ba2I (SEQ ID N°34)** | 60,6 | 56,5 |
| | **2Pa2I (SEQ ID N°35)** | 57,3 | 57,3 |

La sonde molecular beacon CpinfB (SEQ ID N°13), lorsqu'elle est en solution en présence de différentes séquences cibles synthétiques, s'hybride avec elles et produit un signal de fluorescence mesurable. Lorsque la cible synthétique contient des nucléotides complémentaires de la séquence d'un des brins de la partie de la « tige » du molecular beacon CPinfB, on observe une augmentation de la Tm-cible de la sonde molecular beacon. Cette augmentation est proportionnelle au nombre de nucléotides complémentaires de la séquence d'un des brins de la partie tige du molecular beacon, et indique qu'il y a appariement de bases entre les nucléotides de la séquence d'un des brins de la partie « tige » de la sonde CPinfB et les nucléotides complémentaires qui se trouvent adjacents à la séquence d'hybridation avec la cible.

Lorsque la sonde selon l'invention Opa6d (SEQ ID N°1) (sonde de type *alpha)* est en solution en présence de différentes séquences cibles synthétiques, elle s'hybride avec elles et produit un signal de fluorescence mesurable. Lorsque la cible synthétique contient des nucléotides complémentaires d'une des séquences de fermeture de la sonde selon l'invention Opa6d, on observe que les valeurs de Tm-cible de la sonde selon l'invention restent stable, quelque soit le nombre de ces nucléotides complémentaires. Ceci indique qu'il n'y a pas d'appariement de bases entre les nucléotides d'une des séquences de fermeture de la sonde selon l'invention et les nucléotides complémentaires qui se trouvent adjacents à la séquence hybridation avec la cible.

### Conclusion :

La sonde selon l'invention Opa6d est plus spécifique que la sonde molecular beacon CPinfB car il n'y a pas d'interactions supplémentaires entre les nucléotides d'une des séquences de fermeture de la sonde et la séquence de la cible même si ces nucléotides sont complémentaires entre eux. La seule partie de la sonde selon l'invention qui s'hybride avec l'acide nucléique cible correspondant à la séquence de reconnaissance moléculaire de la sonde Opa6d. Le design de cette sonde diminue fortement les possibilités d'hybridation non spécifique de la sonde selon l'invention avec des séquences proches de la séquence de la cible.

### Exemple 10 : Etude de l'hybridation des sondes spécifiques selon l'invention de type alpha en 5'du modèle HIV avec leur séquence cible.

### Objectifs :

L'objectif poursuivi dans cet exemple est le même que celui déjà décrit à l'exemple 9 sur un modèle de cible différent.

### Design expérimental :

Les sondes utilisées sont les suivantes :
Sonde HOa8 (SEQ ID N°2) :
   5'- accctatctc(dT-FAM)CCATCAATGAGGAAGCTGCAGAATGGGATAGAG -3' Da
Molecular beacon HIV-1A (SEQ ID N°21) :
   5'-(FAM)CTATCCCATCAATGAGGAAGCTGCAGAATGGGATAG- 3' Da

Les segments soulignés correspondent aux séquences de fermeture des sondes selon l'invention ou aux brins de la partie « tige » du molecular beacon.

Les nucléotides écrits en minuscule dans les sondes correspondent à des nucléotides de type *alpha.*

Les séquences cibles synthétiques utilisées sont les suivantes :
Cible BioH (SEQ ID N°36) :
   TTTCATTCTGCAGCTTCCTCATTCATTGGTCTCTTTTAAC
Cible 5BH (SEQ ID N°37) :
   TTTCATTCTGCAGCTTCCTCATTGATGGGATAGTTTAAC
Cible 11PH (SEQ ID N°38) :
   TTTCATTCTGCAGCTTCCTCATTGATGGAGAGATAGGGT
Cible 5PH (SEQ ID N°39) :
   TTTCATTCTGCAGCTTCCTCATTGATGGAGAGATTTAAC
Cible 1BH (SEQ ID N°40):
   TTTCATTCTGCAGCTTCCTCATTGATGGGTCTCTTTTAAC
Cible 1PH (SEQ ID N°41) :
   TTTCATTCTGCAGCTTCCTCATTGATGGACTCTTTTAAC
Cible 2BH (SEQ ID N°42) :
   TTTCATTCTGCAGCTTCCTCATTGATGGGATCTTTTAAC
Cible 2PH (SEQ ID N°43) :
   TTTCATTCTGCAGCTTCCTCATTGATGGAGTCTTTTAAC

La séquence soulignée avec des pointillés correspond à la partie hybridable avec la séquence de reconnaissance soit du molecular beacon HIV-1A (SEQ ID N°21) soit de la sonde selon l'invention HOa8 (SEQ ID N°2).

La séquence cible BioH (SEQ ID N°36) correspond à un fragment de la séquence cible biologique du virus HIV. Elle peut contenir un nucléotide complémentaire d'une séquence de fermeture des sondes selon l'invention ou d'une des séquences des brins de la partie « tige » du molecular beacon.

La séquence cible 5BH (SEQ ID N°37) contient, par rapport à la séquence cible BioH (SEQ ID N°36), cinq nucléotides en 3' de la séquence de la séquence soulignée avec des pointillés. Ces cinq nucléotides, soulignés d'un double-trait, sont complémentaires de la séquence de l'un des brins de la partie « tige » du molecular beacon HIV-1A (SEQ ID N°21).

La séquence cible 11PH (SEQ ID N°38) contient, par rapport à la séquence cible BioH (SEQ ID N°36), onze nucléotides en 3' de la séquence surlignée avec des pointillés. Ces onze nucléotides, soulignés d'un double-trait, sont complémentaires d'une des séquences de fermeture de la sonde selon l'invention HOa8 (SEQ ID N°2).

La séquence cible 5PH (SEQ ID N°39) contient, par rapport à la séquence cible BioH (SEQ ID N°36), cinq nucléotides en 3' de la séquence soulignée avec des pointillés. Ces cinq nucléotides, soulignés d'un double-trait, sont complémentaires d'une des séquences de fermeture de la sonde selon l'invention HOa8 (SEQ ID N°2).

La séquence cible 1BH (SEQ ID N°40) contient, par rapport à la séquence cible BioH (SEQ ID N°36), un nucléotide en 3' de la séquence soulignée avec des pointillés. Ce nucléotide, souligné d'un double-trait, est complémentaire de la séquence de l'un des brins de la partie «tige » du molecular beacon HIV-1A (SEQ ID N°21).

La séquence cible 1PH (SEQ ID N°41) contient, par rapport à la séquence cible BioH (SEQ ID N°36), un nucléotide en 3' de la séquence soulignée avec des pointillés. Ce nucléotide, souligné d'un double-trait, est complémentaire d'une des séquences de fermeture de la sonde selon l'invention HOa8 (SEQ ID N°2).

La séquence cible 2BH (SEQ ID N°42) contient, par rapport par rapport à la séquence cible BioH (SEQ ID N°36), deux nucléotides en 3' de la séquence soulignée avec des pointillés. Ces nucléotides, soulignés d'un double-trait, sont complémentaires de la séquence de l'un des brins de la partie «tige » du molecular beacon HIV-1A (SEQ ID N°21).

La séquence cible 2PH (SEQ ID N°43) contient, par rapport à la séquence cible BioH (SEQ ID N°36), deux nucléotides en 3' de la séquence soulignée avec des pointillés. Ces deux nucléotide, soulignés d'un double-trait, sont complémentaires d'une des séquences de fermeture de la sonde selon l'invention HOa8 (SEQ ID N°2).

Les sondes sont introduites dans une solution contenant :
- Diluant basic kit (bioMérieux B.V., Boxtel, Pays Bas, ref. 70352),
- Sonde à concentration 0,1 micromolaire,
- Séquence cible synthétique à concentration 1,25 micromolaire.

Le volume final est de 20 µl.

Des contrôles négatifs sont faits avec de l'eau ultrapure MilliQ à la place de la séquence cible. Cela permet d'observer le comportement de la sonde seule.

Les solutions sont chauffées à 68°C dans le lecteur de fluorescence (EasyQ reader, bioMérieux B.V., Boxtel, Pays Bas) et laissées refroidir pendant 5 heures. La fluorescence de chaque solution est mesurée à 520 nm tout au long du refroidissement. La stabilité thermique de la double hélice sonde/cible est donnée par la valeur de « Tm-cible » qui correspond au point d'inflexion de la courbe fluorescence = f(température). La Tm-cible est calculée comme le minimum de la première dérivée de cette courbe.

### Résultats :

Les résultats sont présentés dans le tableau 6 et dans la figure 8. Le tableau 6 ci-dessous réunit les valeurs de Tm-cible (en °C) obtenues avec les sondes et cibles décrites dans cet exemple. L'augmentation de la Tm-cible par rapport à une Tm-cible de référence indique la formation d'interactions supplémentaires entre la cible et sa sonde. Dans cet exemple, les Tm-cible de référence correspondent aux Tm-cible mesurées entre le molecular beacon HIV-1A (SEQ ID N°21) et la cible BioH (SEQ ID N°36) et celle mesurée entre la sonde selon l'invention Hoa8 (SEQ ID N°2) et la cible BioH (SEQ ID N°36). Elles sont respectivement égale à 60,6°C et 60,2°C.

**Tableau 6 : Présentation des valeurs de Tm-cible obtenues avec les différentes sondes.**

| | | **Sondes** | |
|---|---|---|---|
| | | **HIV-1A** (SEQ ID N°21) | **HOa8** (SEQ ID N°2) |
| **Séquences cible** | **BioH** (SEQ ID N°36) | 60,6 | 60,2 |
| | **5BH** (SEQ ID N°37) | 68* | 60,8 |
| | **11PH** (SEQ ID N°38) | 60,8 | 60,3 |
| | **5PH (SEQ ID N°39)** | 61,5 | 60,8 |
| | **1BH** (SEQ ID N°40) | 61,4 | NA |
| | **1PH** (SEQ ID N°41) | 61,5 | 60,9 |
| | **2BH** (SEQ ID N°42) | 63 | 61,3 |
| | **2PH** (SEQ ID N°43) | 61,6 | 61,3 |

| | | | |
|---|---|---|---|
| * : La valeur de Tm entre le molecular beacon HIV-1A (SEQ ID N°21) et la cible complémentaire 5BH (SEQ ID N°37) était plus haute que la valeur maximale mesurable de 68°C. Pour le besoin de l'expérience, nous avons indiqué la valeur maximale mesurable possible. NA : Non Applicable. La valeur de Tm entre la sonde selon l'invention HOa8 (SEQ ID N°2) et la cible complémentaire 1BH (SEQ ID N°40) n'a pas pu être mesurée car le profil ne correspond pas au standard mesurable avec notre méthode. Cette valeur a été déterminée à partir d'une lecture empirique du graphique et est égale à est de 60°C. | | | |

La sonde molecular beacon HIV-1A (SEQ ID N°21), lorsqu'elle est en solution en présence de différentes séquences cibles synthétiques, s'hybride avec elles et produit un signal de fluorescence mesurable. Lorsque la cible synthétique contient des nucléotides complémentaires de la séquence d'un des brins de la partie de la « tige » du molecular beacon HIV-1A, on observe une augmentation de la Tm-cible de la sonde molecular beacon. Cette augmentation est proportionnelle au nombre de nucléotides complémentaires de la séquence d'un des brins de la partie « tige », et indique qu'il y a appariement de bases entre ces nucléotides et les nucléotides complémentaires qui se trouvent adjacents dans la séquence d'hybridation avec la cible.

Lorsque la sonde selon l'invention HOa8 (SEQ ID N°2) (de type *alpha)*, lorsqu'elle est en solution en présence de différentes séquences cibles synthétiques, s'hybride avec ces séquences et produit un signal de fluorescence mesurable. Lorsque la cible synthétique contient des nucléotides complémentaires d'une des séquences de fermeture de la sonde HOa8, la Tm-cible de la sonde selon l'invention de type *alpha* reste stable, indiquant qu'il n'y a pas d'appariement de bases entre les nucléotides d'une des séquences de fermeture de la sonde selon l'invention et les nucléotides complémentaires qui se trouvent adjacents dans la séquence d'hybridation avec la cible.

### Conclusion :

La sonde selon l'invention HOa8 est plus spécifique que la sonde molecular beacon HIV-1A car il n'y a pas d'interactions supplémentaires entre les nucléotides d'une des séquence de fermeture de la sonde et la séquence de la cible même si ces nucléotides sont complémentaires entre eux. La seule partie de la sonde selon l'invention qui s'hybride avec l'acide nucléique cible correspondant à la séquence de reconnaissance moléculaire de la sonde HOa8. Le design de cette sonde diminue fortement les possibilités d'hybridation non spécifiques de la sonde selon l'invention avec des séquences proches de la séquence de la cible.

### Exempte 11 : Etude de l'hybridation spécifique des sondes selon l'invention de type inversé en 3' du modèle influenza B avec leur séquence cible.

### Objectifs :

L'objectif poursuivi dans cet exemple est le même que celui décrit dans l'exemple 9 mais avec un design de sonde selon l'invention qui est différent.

### Design expérimental :

Les sondes utilisées sont les suivantes :
Sonde Opi6d (SEQ ID N°5) :
   5'- Da -GGAGAAGACGTCCAAAAACTGGCAGA-3'(dT-FAM) 3'-**cctcttctg**-5'
Molecular beacon CP2infB (SEQ ID N°13) :
   5'- CGATCGGGAGAAGACGTCCAAAAACTGGCAGACGATCG -3'Da

Les segments soulignés correspondent aux séquences de fermeture des sondes selon l'invention ou aux brins de la partie « tige » du molecular beacon.

Les nucléotides écrits en minuscule et en gras dans les sondes correspondent à des nucléotides de type *inversé.*

Les séquences cible synthétiques utilisées sont les suivantes :
Cible BioiI (SEQ ID N°44) :
   TTGCAGCTCTTCTGCCAGTTTTTGGACGTCTTCTCCTTT
Cible 6BiI (SEQ ID N°45) :
   TTGCCGATCGTCTGCCAGTTTTTGGACGTCTTCTCCTTT
Cible 10PiI (SEQ ID N°46) :
   CAGAAGAGGATCTGCCAGTTTTTGGACGTCTTCTCCTTT
Cible 6PiI (SEQ ID N°47) :
   TTGCAGAGGATCTGCCAGTTTTTGGACGTCTTCTCCTTT
Cible 1BiI (SEQ ID N°48) :
   TTGCAGCTCGTCTGCCAGTTTTTGGACGTCTTCTCCTTT
Cible 1PiI (SEQ ID N°49) :
   TTGCAGCTCATCTGCCAGTTTTTGGACGTCTTCTCCTTT

La séquence soulignée avec des pointillés correspond à la partie hybridable avec la séquence de reconnaissance soit du molecular beacon CP2infB (SEQ ID N°13) ou soit de la sonde selon l'invention HOa8 (SEQ ID N°5).

La séquence cible BioiI (SEQ ID N°44) correspond à un fragment de la séquence cible biologique du virus Influenza B. Elle peut contenir un nucléotide complémentaire d'une séquence de fermeture des sondes selon l'invention ou d'une séquences des brins de la partie « tige » du molecular beacon.

La séquence cible 6BiI (SEQ ID N°45) contient, par rapport à la séquence cible BioiI (SEQ ID N°44), six nucléotides en 5' de la séquence soulignée avec des pointillés. Ces six nucléotides, soulignés d'un double trait, sont complémentaires de la séquence d'un des brins de la partie « tige » du molecular beacon CP2infB (SEQ ID N°13).

La séquence cible 10PiI (SEQ ID N°46) contient, par rapport à la séquence cible BioiI (SEQ ID N°44), dix nucléotides en 5' de la séquence soulignée avec des pointillés. Ces dix nucléotides, soulignés d'un double trait, sont complémentaires d'une des séquence de fermeture de la sonde selon l'invention Opi6d (SEQ ID N°5).

La séquence cible 6PiI (SEQ ID N°47) contient, par rapport à la séquence cible BioiI (SEQ ID N°44), six nucléotides en 5' de la séquence soulignée avec des pointillés. Ces six nucléotides, soulignés d'un double trait,sont complémentaires de la séquence d'une des séquence de fermeture de la sonde selon l'invention Opi6d (SEQ ID N°5).

La séquence cible 1BiI (SEQ ID N°48) contient, par rapport à la séquence cible BioiI (SEQ ID N°44), un nucléotide en 5' de la séquence soulignée avec des pointillés. Ce nucléotide, souligné d'un double trait,est complémentaire de la séquence d'un des brins de la partie « tige » du molecular beacon CP2infB (SEQ ID N°13).

La séquence cible 1PiI (SEQ ID N°49) contient, par rapport à la séquence cible BioiI (SEQ ID N°44), un nucléotide en 5' de la séquence soulignée avec des pointillés. Ce nucléotide, souligné d'un double trait,est complémentaire d'une des séquence de fermeture de la sonde selon l'invention Opi6d (SEQ ID N°5).

Les sondes sont introduites dans une solution contenant :
- Diluant basic kit (bioMérieux B.V., Boxtel, Pays Bas, ref. 70352),
- Sonde à concentration 0,1 micromolaire,
- Séquence cible synthétique à concentration 1,25 micromolaire.

Le volume final est de 20 µl.

Des contrôles négatifs sont faits avec de l'eau ultrapure type MilliQ à la place de la séquence cible. Cela permet d'observer le comportement de la sonde seule.

Les solutions sont chauffées à 68°C dans le lecteur de fluorescence (EasyQ reader, bioMérieux B.V, Boxtel, Pays Bas) et laissées refroidir pendant 5 heures. La fluorescence de chaque solution est mesurée à 520 nm tout au long du refroidissement. La stabilité thermique de la double hélice sonde/cible est donnée par la valeur de «Tm-cible» qui correspond au point d'inflexion de la courbe. La Tm-cible est calculée comme le minimum de la première dérivée de la courbe.

### Résultats :

Les résultats sont présentés dans le tableau 7 et dans la figure 9. Le tableau 7 réunit les valeurs de Tm-cible (en °C) obtenues avec les sondes et cibles décrites dans cet exemple. L'augmentation de la Tm-cible par rapport à une Tm-cible de référence indique la formation d'interactions supplémentaires entre la cible et sa sonde. Dans cet exemple, les Tm-cible de référence correspondent aux Tm-cible mesurées entre le molecular beacon CP2infB (SEQ ID N°13) et la cible BioiI (SEQ ID N°44) et à celle mesurée entre la sonde selon l'invention Opi6d (SEQ ID N°5) et la cible BioiI (SEQ ID N°44). Elles sont respectivement égale à 62°C et 62,9°C.

**Tableau 7 : Présentation des valeurs de Tm-cible obtenues avec les différentes sondes.**

| | | **Sondes** | |
|---|---|---|---|
| | | **CP2infB** (SEQ ID N°13) | **OPi6d** (SEQ ID N°5) |
| **Séquences cible** | **BioiI** (SEQ ID N°44) | 62 | 62,9 |
| | **6BiI** (SEQ ID N°45) | 68* | 64,5 |
| | **10PiI** (SEQ ID N°46) | 62,8 | 64,4 |
| | **6PiI** (SEQ ID N°47) | 62,4 | 63,4 |
| | **1BiI** (SEQ ID N°48) | 64,7 | 63,9 |
| | **1PiI** (SEQ ID N°49) | 61,9 | 62,7 |

| | | | |
|---|---|---|---|
| * : La valeur de Tm entre le molecular beacon CP2infB (SEQ ID N°13) et la cible complémentaire 6BiI (SEQ ID N°45) était plus haute que la valeur maximale mesurable de 68°C. Pour le besoin de l'expérience, nous avons indiqué la valeur maximale mesurable possible. | | | |

La sonde molecular beacon CP2infB (SEQ ID N°13), lorsqu'elle est en solution en présence de différentes séquences cibles synthétiques, s'hybride avec ces séquences cibles synthétiques et produit un signal de fluorescence mesurable. Lorsque la cible synthétique contient des nucléotides complémentaires de la séquence d'un des brins de la partie « tige » de la sonde molecular beacon CP2infB (SEQ ID N°13), on observe une augmentation proportionnelle de la valeur la Tm-cible de la sonde molecular beacon par rapport au nombre de nucléotides complémentaires de la séquence d'un des brins de la partie « tige ». Ceci indique qu'il y a appariement de bases entre les nucléotides d'un des brins de la partie « tige »de la sonde CP2infB (SEQ ID N°13) et les nucléotides complémentaires qui se trouvent adjacents dans la séquence de la cible.

La sonde selon l'invention Opi6d (de type inversé) (SEQ ID N°5), lorsqu'elle est en solution en présence de différentes séquences cibles synthétiques, s'hybride avec ces séquences cibles synthétiques et produit un signal de fluorescence mesurable. Lorsque la cible synthétique contient des nucléotides complémentaires d'une des séquence de fermeture de la sonde selon l'invention Opi6d (SEQ ID N°5), la Tm-cible de la sonde selon l'invention de type *inversé* reste stable, indiquant qu'il n'y a pas d'appariement de bases entre les nucléotides d'une des séquence de fermeture de la sonde et les nucléotides complémentaires qui se trouvent adjacents dans la séquence de la cible.

### Conclusion

La sonde selon l'invention Opi6d est plus spécifique que la sonde molecular beacon CPinfB car il n'y a pas d'interactions supplémentaires entre les nucléotides d'une des séquence de fermeture de la sonde et la séquence de la cible même si ces nucléotides sont complémentaires entre eux. La seule partie de la sonde selon l'invention qui s'hybride avec l'acide nucléique cible correspondant à la séquence de reconnaissance moléculaire de la sonde Opi6d. Le design de cette sonde diminue fortement les possibilités d'hybridation non spécifiques de la sonde selon l'invention avec des séquences proches de la séquence de la cible.

### Exemple 12 : Etude de l'hybridation des sondes selon l'invention de type inversé en 5' du modèle HIV avec leur séquence cible.

### Objectifs :

L'objectif poursuivi dans cet exemple est le même que celui décrit dans l'exemple 9 mais avec un design de sonde selon l'invention et un modèle de cible qui sont différents.

### Design expérimental :

Les sondes utilisées sont les suivantes :
Sonde HOi8 (SEQ ID N°50) :
   3'- **accctatctc**-5'-5'-(dT-FAM)CCATCAATGAGGAAGCTGCAGAATGGGATAGAG-3' Da
Molecular beacon référence HIV-1A (SEQ ID N°21) :
   5'- (FAM)-CTATCCCATCAATGAGGAAGCTGCAGAATGGGATAG -3' Da

Les segments soulignés correspondent aux séquences de fermeture des sondes selon l'invention ou aux brins de la partie « tige » du molecular beacon.

Les nucléotides écrits en minuscule et en gras dans les sondes correspondent à des nucléotides de type *inversé.*

Les séquences cible synthétiques utilisées sont les suivantes :
Cible BioH (SEQ ID N°36) :
   5'- TTTCATTCTGCAGCTTCCTCATTGATGGTCTCTTTTAAC -3'
Cible 5BH (SEQ ID N°37) :
   5'- TTTCATTCTGCAGCTTCCTCATTGATGGGATAGTTTAAC -3'
Cible 11PH (SEQ ID N°38) :
   5'- TTTCATTCTGCAGCTTCCTCATTGATGGAGAGATAGGGT -3'
Cible 5PH (SEQ ID N°39) :
   5'- TTTCATTCTGCAGCTTCCTCATTGATGGAGAGATTTAAC -3'
Cible 1BH (SEQ ID N°40) :
   5'- TTTCATTCTGCAGCTTCCTCATTGATGGGCTCTTTTAAC-3'
Cible 1PH (SEQ ID N°41) :
   5'-TTTCATTCTGCAGCTTCCTCATTGATGGACTCTTTTAAC-3'
Cible 2BH (SEQ ID N°42) :
   5'- TTTCATTCTGCAGCTTCCTCATTGATGGGATCTTTTAAC-3'
Cible 2PH (SEQ ID N°43) :
   5'- TTTCATTCTGCAGCTTCCTCATTGATGGAGTCTTTTAAC-3'

La séquence soulignée avec des pointillés correspond à la partie hybridable avec la séquence de reconnaissance soit du molecular beacon HIV-1A (SEQ ID N°21) soit de la sonde selon l'invention HOi8 (SEQ ID N°50).

La séquence cible BioH (SEQ ID N°36) correspond à un fragment de la séquence cible biologique du virus HIV. Elle peut contenir un nucléotide complémentaire d'une séquence de fermeture des sondes selon l'invention ou d'une séquences des brins de la partie « tige » du molecular beacon.

La séquence cible 5BH (SEQ ID N°37) contient, par rapport à la séquence cible BioH (SEQ ID N°36), cinq nucléotides en 3' de la séquence soulignée avec des pointillés. Ces cinq nucléotides, soulignés d'un double trait, sont complémentaires de la séquence de l'un des brins de la partie « tige » du molecular beacon HIV-1A (SEQ ID N°21).

La séquence cible 11 PH (SEQ ID N°38) contient, par rapport à la séquence cible BioH (SEQ ID N°36), onze nucléotides en 3' de la séquence de la séquence soulignée avec des pointillés. Ces onze nucléotides, soulignés d'un trait double, sont complémentaires d'une des séquences de fermeture de la sonde selon l'invention HOi8 (SEQ ID N°50).

La séquence cible 5PH (SEQ ID N°39) contient, par rapport à la séquence cible BioH (SEQ ID N°36), cinq nucléotides en 3' de la séquence de la séquence soulignée avec des pointillés.

Ces cinq nucléotides, soulignés d'un trait double, sont complémentaires d'une des séquences de fermeture de la sonde selon l'invention HOi8 (SEQ ID N°50).

La séquence cible 1BH (SEQ ID N°40) contient, par rapport à la séquence cible BioH (SEQ ID N°36), un nucléotide en 3' de la séquence soulignée avec des pointillés. Ce nucléotide, souligné d'un double trait, est complémentaire de la séquence de l'un des brins de la partie « tige » du molecular beacon HIV-1A (SEQ ID N°21).

La séquence cible 1PH (SEQ ID N°41) contient, par rapport à la séquence cible BioH (SEQ ID N°36), un nucléotide en 3' de la séquence de la séquence soulignée avec des pointillés. Ce nucléotide souligné, d'un trait double,est complémentaire d'une des séquences de fermeture de la sonde selon l'invention HOi8 (SEQ ID N°50).

La séquence cible 2BH (SEQ ID N°42) contient, par rapport à la séquence cible BioH (SEQ ID N°36), deux nucléotides en 3' de la séquence soulignée avec des pointillés. Ces deux nucléotides, soulignés d'un double trait, sont complémentaires de la séquence de l'un des brins de la partie « tige » du molecular beacon HIV-1A (SEQ ID N°21).

La séquence cible 2PH (SEQ ID N°43) contient, par rapport à la séquence cible BioH (SEQ ID N°36), deux nucléotides en 3' de la séquence de la séquence soulignée avec des pointillés. Ces deux nucléotides, soulignés d'un trait double,sont complémentaires d'une des séquences de fermeture de la sonde selon l'invention HOi8 (SEQ ID N°50).

Les sondes sont introduites dans une solution contenant :
- Diluant basic kit (bioMérieux B.V., Boxtel, Pays Bas, ref. 70352),
- Sonde à concentration 0,1 micromolaire,
- Séquence cible synthétique à concentration 1,25 micromolaire.

Le volume final est de 20 µl.

Des contrôles négatifs sont faits avec de l'eau ultrapure type MilliQ à la place de la séquence cible. Cela permet d'observer le comportement de la sonde seule.

Les solutions sont chauffées à 68°C dans le lecteur de fluorescence (EasyQ reader, bioMérieux B.V., Boxtel, Pays Bas) et laissées refroidir pendant 5 heures. La fluorescence de chaque solution est mesurée à 520 nm tout au long du refroidissement. La stabilité thermique de la double hélice sonde/cible est donnée par la valeur de « Tm-cible» qui correspond au point d'inflexion de la courbe. La Tm-cible est calculée comme le minimum de la première dérivée de la courbe.

### Résultats :

Les résultats sont présentés dans le tableau 8 et la figure 10. Le tableau 8 réunit les valeurs de Tm-cible (en °C) obtenues avec les sondes et cibles décrites dans cet exemple. L'augmentation de la Tm-cible par rapport à une Tm-cible de référence indique la formation d'interactions supplémentaires entre la cible et sa sonde. Dans cet exemple, les Tm-cible de référence correspondent aux Tm-cible mesurées entre le molecular beacon HIV-1A (SEQ ID N°21) et la cible BioH (SEQ ID N°36) et à celle mesurée entre la sonde selon HOi8 (SEQ ID N°50) et la cible BioH (SEQ ID N°36). Elles sont respectivement égales à 60,6°C et 61,1°C.

**Tableau 8 : Présentation des valeurs de Tm-cible obtenues avec les différentes sondes.**

| | | **Sondes** | |
|---|---|---|---|
| | | **HIV-1A** (SEQ ID N°21) | **HOi8** (SEQ ID N°50) |
| **Sequences cible** | **BioH** (SEQ ID N°36) | 60,6 | 61,1 |
| | **5BH** (SEQ ID N°37) | 68* | 60,7 |
| | **11PH** (SEQ ID N°38) | 60,8 | 61,3 |
| | **5PH** (SEQ ID N°39) | 61,5 | 62,2 |
| | **1BH** (SEQ ID N°40) | 61,4 | 60,6 |
| | **1PH** (SEQ ID N°41) | 61,5 | 60,9 |
| | **2BH** (SEQ ID N°42) | 63 | 62,3 |
| | **2PH** (SEQ ID N°43) | 61,6 | 62,3 |

| | | | |
|---|---|---|---|
| * : La valeur de Tm entre le molecular beacon HIV-1A et la cible complémentaire 5BH était plus haute que la valeur maximale mesurable de 68°C. Pour le besoin de l'expérience, nous avons indiqué la valeur maximale mesurable possible. | | | |

La sonde molecular beacon HIV-1A (SEQ ID N°21), lorsqu'elle est en solution en présence de différentes séquences cibles synthétiques, s'hybride avec ces séquences cibles synthétiques et produit un signal de fluorescence mesurable. Lorsque la cible synthétique contient des nucléotides complémentaires de la séquence d'un des brins de la partie « tige » du molecular beacon HIV-1A (SEQ ID N°21), on observe que la Tm-cible de la sonde molecular beacon augmente de façon proportionnelle par rapport au nombre de nucléotides complémentaires de la séquence d'un des brins de la partie « tige ». Ceci indique qu'il y a appariement de bases entre les nucléotides de la séquence d'un des brins de la partie « tige » de la sonde HIV-1A (SEQ ID N°21) et les nucléotides complémentaires qui se trouvent adjacents dans la séquence de la cible.

La sonde selon l'invention HOi8 (de type inversé) (SEQ ID N°50), lorsqu'elle est en solution en présence de différentes séquences cibles synthétiques, s'hybride avec ces séquences cibles synthétiques et produit un signal de fluorescence mesurable. Lorsque la cible synthétique contient des nucléotides complémentaires d'une des séquences de fermeture de la sonde selon l'invention HOi8 (SEQ ID N°50), la Tm-cible de la sonde selon l'invention de type inversé reste stable, indiquant qu'il n'y a pas d'appariement de bases entre les nucléotides d'une des séquences de fermeture de la sonde selon l'invention et les nucléotides complémentaires qui se trouvent adjacents dans la séquence de la cible.

### Conclusion :

La sonde selon l'invention Hoi8 est plus spécifique que la sonde molecular beacon HIV-1A car il n'y a pas d'interactions supplémentaires entre les nucléotides d'une des séquence de fermeture de la sonde et la séquence de la cible même si ces nucléotides sont complémentaires entre eux. La seule partie de la sonde selon l'invention qui s'hybride avec l'acide nucléique cible correspondant à la séquence de reconnaissance moléculaire de la sonde Hoi8. Le design de cette sonde diminue fortement les possibilités d'hybridation non spécifiques de la sonde selon l'invention avec des séquences proches de la séquence de la cible.

### Conclusion générale :

Ces exemples démontrent que quelque soit le design de la sonde selon l'invention , cette dernière présente une spécificité d'hybridation supérieure à celle d'une sonde molecular beacon. La nature des nucléotides (nucléotide *alpha* ou nucléotide inversé) ainsi que leur position par rapport au deuxième fragment de la sonde selon l'invention (position en 3' ou position en 5') n'influence pas la qualité du design ni la spécificité d'hybridation de la sonde selon l'invention pour son acide nucléique cible.

### Exemple 13 : Etude de la diminution du signal non spécifique d'origine enzymatique par l'utilisation des sondes selon l'invention.

### Objectifs :

L'objectif de cet exemple est d'étudier la sensibilité des sondes selon l'invention par rapport aux phénomènes d'ouverture prématurée non désirées desdites sondes.

### Design expérimental :

Les sondes sont incubées ensemble avec l'enzyme T7 ARN polymérase utilisée dans la NASBA dans le mélange réactionnel, dont la composition est décrite ci-dessous, pendant quatre heures à 41°C, dans le lecteur de fluorescence (EasyQ reader, bioMérieux B.V., Boxtel, Pays Bas). La composition du mélange réactionnel est:
- 10µL de « reagent mix », préparé suivant les instructions du fournisseur à partir d'une NucliSens EQ Basic Kit reagent sphere (bioMérieux B.V., Boxtel, NL, art. nr. 60085110) diluée dans 64 microlitres de Basic Kit reagent sphere diluent (bioMérieux B.V., Boxtel, NL, art. nr. 70352),
- 3,76 microlitres de sorbitol (bioMérieux B.V., Boxtel, NL, art. nr. 60085192),
- 0,2 microlitres d'enzyme T7 ARN Polymerase à une concentration de 80 unités par microlitre (bioMérieux B.V., Boxtel, NL, art. nr. 72236),
- 2 microlitres de sonde à une concentration initiale de 2 micromolaire.
- Quantité suffisante de solution « enzyme mix solution » pour un volume final de 20 microlitres.

Un mélange réactionnel de référence est réalisé qui contient tous les composants sauf l'enzyme, laquelle est remplacée par un volume équivalent de « enzyme mix solution ».

La solution « enzyme mix solution » est préparée en réalisant un mélange de plusieurs autres solutions, comme suit :
80 microlitres de RT storage buffer (bioMérieux B.V., Boxtel, NL, art. nr. 60085341),
114 microlitres de T7 storage buffer (bioMérieux B.V., Boxtel, NL, art. nr. 60085337),
20 microlitres de RH storage buffer (bioMérieux B.V., Boxtel, NL, art. nr. 60085339),
411 micro litres de Premix solution (bioMérieux B.V., Boxtel, NL, art. nr. 60085335),
625 micro litres d'eau ultra ultrapure type MilliQ.

Le signal de fluorescence est enregistré pendant quatre heures à 41°C, à la longueur d'onde d'émission de la fluorescéine.

Les sondes utilisées sont les suivantes :
Sonde Opi6d (SEQ ID N°5) :
   5'- Da -GGAGAAGACGTCCAAAAACTGGCAGA- 3'(dT-FAM) 3'-**cctcttctg**-5'
Sonde Opa6d (SEQ ID N°1) :
   5'-gaccgctg(dT-FAM)GGAGAAGACGTCCAAAAACTGGCAGAC- 3' Da
Sonde HOi8 (SEQ ID N°50) :
   3'-**accctatctc**-5'-5'-(dT-FAM)CCATCAATGAGGAAGCTGCAGAATGGGATAGAG-3' Da
Sonde HOa8 (SEQ ID N°2) :
   5'- accctatctc(dT-FAM)CCATCAATGAGGAAGCTGCAGAATGGGATAGAG -3' Da
Molecular beacon HIV-1A (SEQ ID N°21) :
   5'- (FAM)-CTATCCCATCAATGAGGAAGCTGCAGAATGGGATAG -3' Da
Molecular beacon CpinfB (SEQ ID N°13) :
   5'- (FAM)-CGATCGGGAGAAGACGTCCAAAAACTCGATCG -3'Da

Les séquences nucléotidiques soulignées correspondent aux séquences de fermeture des sondes selon l'invention ou aux séquences des brins formant la partie « tige » du molecular beacon.

Les nucléotides écrits en minuscule dans les sondes correspondent à des nucléotides de type *alpha.* Les nucléotides écrits en minuscule et en gras dans les sondes correspondant à des nucléotides inversés.

### Résultats :

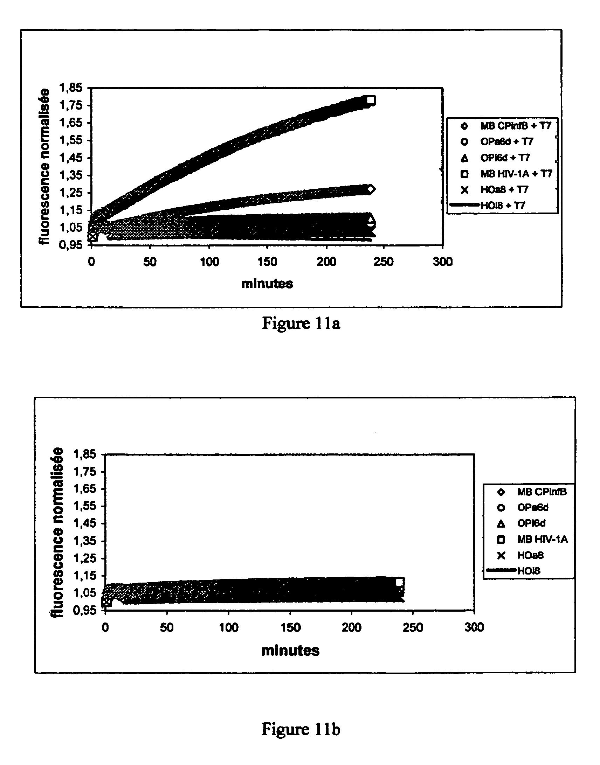

L'émission de fluorescence est mesurée lors de l'incubation des différentes sondes en présence (figure 11a) ou en l'absence (figure 11b) d'enzyme T7 ARN polymérase.

En absence d'acide nucléique cible et en absence de T7 ARN polymérase (figure 11b), aucun signal de fluorescence n'est détecté, que ce soit pour les sondes selon l'invention ou pour les sondes molecular beacons. Ceci signifie que dans ces conditions expérimentales, le fluorophore et le quencheur sont à une distance proche l'un de l'autre permettant le « quenching » de la fluorescence. Les sondes molecular beacon sont donc dans une conformation fermée, c'est à dire ont une structure secondaire en forme d'épingle à cheveux. Les sondes selon l'invention sont dans une conformation fermée, conformation dite circulaire.

En présence de la T7 ARN polymérase et en l'absence de tout acide nucléique cible, la fluorescence émise par les sondes selon l'invention est la même que celle mesurée en l'absence de la T7 ARN polymérase et en l'absence de tout acide nucléique cible. On observe ici aussi un « quenching » de fluorescence. Les sondes selon l'invention sont toujours dans leur conformation initiale, c'est à dire dans une conformation circulaire même en présence de l'enzyme.

En revanche, lorsque de l'enzyme T7 ARN polymérase est ajouté dans le milieu réactionnel contenant les sondes molecular beacon, on observe une augmentation du signal de fluorescence. Cela signifie que la distance entre le fluorophore et le quencheur a augmenté. Un changement de conformation du molecular beacon s'est opéré lors de l'ajout de la T7 ARN polymérase. Les sondes molecular beacons se sont ouvertes. Elles ont une conformation linéaire comme si elles s'étaient hybridées à leur acide nucléique cible.

### Conclusion :

Les résultats montrent que les deux molecular beacons analysés, CPinfB et HIV-1A, produisent une augmentation du signal lors de leur incubation en présence de l'enzyme T7 ARN Polymérase, alors que les sondes selon l'invention analysées dans les mêmes conditions ne produisent pas cette même augmentation du signal. Ce résultat se vérifie pour toutes les sondes selon l'invention testées, qu'elles aient une séquence de fermeture modifiée en 3' ou en 5', et qu'elles contiennent des nucléotides *alpha* ou inversés. Les sondes selon l'invention ne sont donc pas sensibles aux effets des contaminants tels que la T7 ARN polymérase par exemple. Il n'y a pas d'ouverture spontanée non désirée des sondes selon l'invention, donc pas d'émission d'un signal non spécifique.

## Revendications

1. Sonde de détection d'un acide nucléique cible, constituée d'un brin nucléotidique marqué comportant trois fragments :
- un premier fragment présentant une première séquence de fermeture,
- un deuxième fragment dont tout ou partie présente une séquence de reconnaissance destinée à la reconnaissance moléculaire de l'acide nucléique cible,
- un troisième fragment présentant une deuxième séquence de fermeture, et
- au moins deux marqueurs, une des extrémités du brin de ladite sonde de détection étant libre de tout marqueur,
dans laquelle, lorsque les deux séquences de fermeture sont hybridées ensemble, la sonde de détection a une forme complètement circulaire, lesdites séquences de fermeture maintenant ainsi ladite sonde dans une conformation non détectable en l'absence dudit acide nucléique.

2. Sonde de détection selon la revendication 1 **caractérisée en ce qu'**un premier marqueur est porté par un nucléotide du premier fragment et un second marqueur est porté par un nucléotide du deuxième ou du troisième fragment, les deux nucléotides étant avoisinants lorsque les deux séquences de fermeture sont hybridées.

3. Sonde de détection, selon l'une quelconque des revendications 1 ou 2, **caractérisée en ce que** tout ou partie des deux séquences de fermeture s'hybrident de façon parallèle.

4. Sonde de détection, selon l'une quelconque des revendications 1 à 3, **caractérisée en ce que** tout ou partie de l'une des séquences de fermeture est une séquence constituée de nucléotides *alpha.*

5. Sonde de détection, selon l'une quelconque des revendications 1 ou 2, **caractérisée en ce que** tout ou partie des deux séquences de fermeture s'hybrident de façon antiparallèle.

6. Sonde de détection, selon l'une quelconque des revendications 1, 2 ou 5, **caractérisée en ce qu'**une des deux séquences de fermeture est liée à la séquence de reconnaissance par une liaison inversée 5'-5' ou 3'-3'.

7. Sonde de détection, selon l'une quelconque des revendications 1 à 6, **caractérisée en ce qu'**au moins un marqueur est un fluorophore et au moins un autre marqueur est un extincteur de fluorescence.

8. Sonde, selon l'une quelconque des revendications 1 à 7, **caractérisée en ce que** tout ou partie de la première ou de la seconde séquence de fermeture peut s'hybrider sur la séquence cible.

9. Sonde, selon l'une quelconque des revendications 1 à 8, **caractérisée en ce qu'**une séquence nucléotidique est attachée à l'extrémité de l'une des séquences de fermeture.

10. Méthode pour détecter une séquence cible susceptible d'être présente dans un échantillon comprenant les étapes suivantes :
- mettre en contact l'échantillon avec une sonde de détection selon l'une quelconque des revendications précédentes,
- détecter la formation d'un hybride entre la sonde de détection et la séquence cible, indiquant la présence de ladite séquence cible dans ledit échantillon.

11. Méthode selon la revendication 10, **caractérisée en ce que** la détection se fait conjointement à une réaction d'amplification de la séquence cible.

12. Méthode selon la revendication 10 **caractérisée en ce que** la détection se fait après une réaction d'amplification de la séquence cible.

13. Kit pour détecter une séquence cible comprenant au moins une sonde de détection selon l'une quelconque des revendications 1 à 9.

## Claims

1. Probe for detecting a target nucleic acid, constituted of a labeled nucleotide strand comprising three fragments:
- a first fragment having a first closing sequence,
- a second fragment, all or part of which has a recognition sequence intended for the molecular recognition of the target nucleic acid,
- a third fragment having a second closing sequence, and
- at least two markers, one of the ends of the strand of said detection probe being free of any marker,
in which, when the two closing sequences are hybridized together, the detection probe has a completely circular shape, said closing sequences thus maintaining said probe in a conformation that cannot be detected in the absence of said nucleic acid.

2. Detection probe according to Claim 1, **characterized in that** a first marker is carried by a nucleotide of the first fragment and a second marker is carried by a nucleotide of the second or of the third fragment, the two nucleotides being adjacent when the two closing sequences are hybridized.

3. Detection probe according to either one of Claims 1 or 2, **characterized in that** all or part of the two closing sequences are hybridized in parallel.

4. Detection probe according to any one of Claims 1 to 3, **characterized in that** all or part of one of the closing sequences is a sequence constituted of *alpha* nucleotides.

5. Detection probe according to either one of Claims 1 or 2, **characterized in that** all or part of the two closing sequences undergo antiparallel hybridization.

6. Detection probe according to any one of Claims 1, 2 or 5, **characterized in that** one of the two closing sequences is bound to the recognition sequence by a 5'-5' or 3'-3' inverted bond.

7. Detection probe according to any one of Claims 1 to 6, **characterized in that** at least one marker is a fluorophore and at least one other marker is a fluorescence quencher.

8. Probe according to any one of Claims 1 to 7, **characterized in that** all or part of the first or of the second closing sequence can hybridize to the target sequence.

9. Probe according to any one of Claims 1 to 8, **characterized in that** a nucleotide sequence is attached to the end of one of the closing sequences.

10. Method for detecting a target sequence that may be present in a sample comprising the following stages:
- contacting the sample with a detection probe according to any one of the preceding claims,
- detecting the formation of a hybrid between the detection probe and the target sequence, indicating the presence of said target sequence in said sample.

11. Method according to Claim 10, **characterized in that** detection is carried out jointly with a reaction of amplification of the target sequence.

12. Method according to Claim 10, **characterized in that** detection is carried out after a reaction of amplification of the target sequence.

13. A kit for detecting a target sequence comprising at least one detection probe according to any one of Claims 1 to 9.

## Patentansprüche

1. Sonde zum Nachweis einer Zielnukleinsäure, bestehend aus einem markierten Nukleotidstrang, der drei Fragmente enthält:
- ein erstes Fragment, das eine erste Verschlusssequenz aufweist,
- ein zweites Fragment, das insgesamt oder teilweise eine Erkennungssequenz darstellt, die zur molekularen Erkennung der Zielnukleinsäure dient,
- ein drittes Fragment, das eine zweite Verschlusssequenz aufweist, und
- mindestens zwei Marker, wobei eines der Enden des Strangs der Nachweissonde frei von jeglichem Marker ist,
wobei die Nachweissonde, wenn die beiden Verschlusssequenzen aneinander hybridisiert sind, eine vollständig zirkuläre Form besitzt, so dass die Verschlusssequenzen die Sonde somit in Abwesenheit der Nukleinsäure in einer nicht nachweisbaren Form halten.

2. Nachweissonde nach Anspruch 1, **dadurch gekennzeichnet, dass** ein erster Marker von einem Nukleotid des ersten Fragments getragen wird und ein zweiter Marker von einem Nukleotid des zweiten oder dritten Fragments getragen wird, wobei die beiden Nukleotide benachbart sind, wenn die beiden Verschlusssequenzen hybridisiert sind.

3. Nachweissonde nach einem der Ansprüche 1 oder 2, **dadurch gekennzeichnet, dass** die beiden Verschlusssequenzen vollständig oder teilweise parallel hybridisieren.

4. Nachweissonde nach einem der Ansprüche 1 bis 3, **dadurch gekennzeichnet, dass** eine der Verschlusssequenzen vollständig oder teilweise eine aus *alpha-*Nukleotiden bestehende Sequenz ist.

5. Nachweissonde nach einem der Ansprüche 1 oder 2, **dadurch gekennzeichnet, dass** die beiden Verschlusssequenzen vollständig oder teilweise antiparallel hybridisieren.

6. Nachweissonde nach einem der Ansprüche 1, 2 oder 5, **dadurch gekennzeichnet, dass** eine der beiden Verschlusssequenzen an die Erkennungssequenz durch eine invertierte 5'-5'- oder 3'-3'-Bindung gebunden ist.

7. Nachweissonde nach einem der Ansprüche 1 bis 6, **dadurch gekennzeichnet, dass** mindestens ein Marker ein Fluorophor ist und mindestens ein anderer Marker ein Fluoreszenzlöscher ist.

8. Sonde nach einem der Ansprüche 1 bis 7, **dadurch gekennzeichnet, dass** die erste oder die zweite Verschlusssequenz vollständig oder teilweise an die Zielsequenz hybridisieren kann.

9. Sonde nach einem der Ansprüche 1 bis 8, **dadurch gekennzeichnet, dass** eine Nukleotidsequenz an das Ende einer der Verschlusssequenzen angeheftet ist.

10. Verfahren zum Nachweisen einer Zielsequenz, die in einer Probe vorliegen kann, wobei man in den folgenden Schritten:
- die Probe mit einer Nachweissonde nach einem der vorhergehenden Ansprüche in Kontakt bringt,
- die Bildung eines Hybrids zwischen der Nachweissonde und der Zielsequenz nachweist, was das Vorliegen der Zielsequenz in der Probe anzeigt.

11. Verfahren nach Anspruch 10, **dadurch gekennzeichnet, dass** der Nachweis zusammen mit einer Reaktion zur Amplifikation der Zielsequenz erfolgt.

12. Verfahren nach Anspruch 10, **dadurch gekennzeichnet, dass** der Nachweis nach einer Reaktion zur Amplifikation der Zielsequenz erfolgt.

13. Kit zum Nachweis einer Zielsequenz, das mindestens eine Nachweissonde nach einem der Ansprüche 1 bis 9 umfasst.


### SEQUENCE LISTING

<110> bioMérieux
<120> Nouvelle sonde de détection
<130> O-PROBE
<150> FR0854549
   <151> 2008-07-04
<160> 50
<170> PatentIn version 3.3
<210> 1
   <211> 37
   <212> DNA
   <213> Artificial
<220>
   <223> Probe
<220>
   <221> misc_feature
   <222> (1)..(9)
   <223> nucleic acid with an anomeric alpha configuration
<400> 1
   gaccgtctgt ggagaagacg tccaaaaact ggcagac 37
<210> 2
   <211> 44
   <212> DNA
   <213> Artificial
<220>
   <223> Probe
<220>
   <221> misc_feature
   <222> (1)..(10)
   <223> nucleic acid with an anomeric alpha configuration
<400> 2
   accctatctc tccatcaatg aggaagctgc agaatgggat agag 44
<210> 3
   <211> 26
   <212> DNA
   <213> Artificial
<220>
   <223> Target
<400> 3
   tttagttttt ggacgtcttc tccttt 26
<210> 4
   <211> 38
   <212> DNA
   <213> Artificial
<220>
   <223> Target
<400> 4
   tttactctat cccattctgc agcttcctca ttgatttt 38
<210> 5
   <211> 36
   <212> DNA
   <213> Artificial
<220>
   <223> Probe
<220>
   <221> misc_feature
   <222> (1)..(27)
   <223> natural nucleotides carrying (5'-3') phosphodiester linkages
<220>
   <221> misc_feature
   <222> (28) . . (36)
   <223> inverted nucleotides
<400> 5
   ggagaagacg tccaaaaact ggcagatcct cttctg 36
<210> 6
   <211> 45
   <212> DNA
   <213> Artificial
<220>
   <223> Probe
<220>
   <221> misc_feature
   <222> (1)..(34)
   <223> natural nucleotides carrying (5'-3') phosphodiester linkages
<220>
   <221> misc_feature
   <222> (35)..(45)
   <223> inverted nucleotides
<400> 6
   ccatcaatga ggaagctgca gaatgggata gagtggtagt tactc 45
<210> 7
   <211> 47
   <212> DNA
   <213> Artificial
<220>
   <223> Promoter and primer
<400> 7
   aattctaata cgactcacta taggggagct ctgctttagc acttcca 47
<210> 8
   <211> 24
   <212> DNA
   <213> Artificial
<220>
   <223> Primer
<400> 8
   gagaaatgca gatggtctca gcta 24
<210> 9
   <211> 54
   <212> DNA
   <213> Artificial
<220>
   <223> Primer and promoter
<400> 9
   aattctaata cgactcacta tagggtgcta tgtcacttcc ccttggttct ctca 54
<210> 10
   <211> 29
   <212> DNA
   <213> Artificial
<220>
   <223> Primer
<400> 10
   agtgggggga catcaagcag ccatgcaaa 29
<210> 11
   <211> 40
   <212> DNA
   <213> Artificial
<220>
   <223> Probe
<220>
   <221> misc_feature
   <222> (1)..(9)
   <223> nucleic acid with an anomeric alpha configuration
<400> 11
   accctatcct atcaatgagg aagctgcaga atgggatagg 40
<210> 12
   <211> 43
   <212> DNA
   <213> Artificial
<220>
   <223> Probe
<220>
   <221> misc_feature
   <222> (1)..(33)
   <223> natural nucleotides carrying (5'-3') phosphodiester linkages
<220>
   <221> misc_feature
   <222> (34)..(43)
   <223> inverted nucleotides
<400> 12
   ccatcaatga ggaagctgca gaatgggata ggtggtagtt act 43
<210> 13
   <211> 32
   <212> DNA
   <213> Artificial
<220>
   <223> Probe
<400> 13
   cgatcgggag aagacgtcca aaaactcgat cg 32
<210> 14
   <211> 37
   <212> DNA
   <213> Artificial
<220>
   <223> Probe
<220>
   <221> misc_feature
   <222> (1)..(9)
   <223> nucleic acid with an anomeric alpha configuration
<400> 14
   gagcgtagct ggagaagacg tccaaaaact cgcatcg 37
<210> 15
   <211> 35
   <212> DNA
   <213> Artificial
<220>
   <223> Probe
<220>
   <221> misc_feature
   <222> (1)..(8)
   <223> nucleic acid with an anomeric alpha configuration
<400> 15
   gagctagctg gagaagacgt ccaaaaactc gatcg 35
<210> 16
   <211> 35
   <212> DNA
   <213> Artificial
<220>
   <223> Probe
<220>
   <221> misc_feature
   <222> (1)..(8)
   <223> inverted nucleotides
<220>
   <221> misc_feature
   <222> (9)..(35)
   <223> natural nucleotides carrying (5'-3') phosphodiester linkages
<400> 16
   gagctagctg gagaagacgt ccaaaaactc gatcg 35
<210> 17
   <211> 35
   <212> DNA
   <213> Artificial
<220>
   <223> Probe
<220>
   <221> misc_feature
   <222> (1)..(7)
   <223> inverted nucleotides
<220>
   <221> misc_feature
   <222> (8)..(35)
   <223> natural nucleotides carrying (5'-3') phosphodiester linkages
<400> 17
   gctagcctgg agaagacgtc caaaaactcg atcgg 35
<210> 18
   <211> 26
   <212> DNA
   <213> Artificial
<220>
   <223> Target
<400> 18
   tttagttttt ggaggtcttc tccttt 26
<210> 19
   <211> 26
   <212> DNA
   <213> Artificial
<220>
   <223> Target
<400> 19
   tttagttttt ggaagtcttc tccttt 26
<210> 20
   <211> 26
   <212> DNA
   <213> Artificial
<220>
   <223> Target
<400> 20
   tttagttttt ggatgtcttc tccttt 26
<210> 21
   <211> 36
   <212> DNA
   <213> Artificial
<220>
   <223> Probe
<400> 21
   ctatcccatc aatgaggaag ctgcagaatg ggatag 36
<210> 22
   <211> 39
   <212> DNA
   <213> Artificial
<220>
   <223> Probe
<220>
   <221> misc_feature
   <222> (1)..(10)
   <223> nucleic acid with an anomeric alpha configuration
<400> 22
   ttaccctatc ttcaatgagg aagctgcaga atgggatag 39
<210> 23
   <211> 44
   <212> DNA
   <213> Artificial
<220>
   <223> Probe
<220>
   <221> misc_feature
   <222> (1)..(33)
   <223> natural nucleotides carrying (5'-3') phosphodiester linkages
<220>
   <221> misc_feature
   <222> (34)..(44)
   <223> inverted nucleotides
<400> 23
   ccatcaatga ggaagctgca gaatgggata ggtggtagtt actc 44
<210> 24
   <211> 24
   <212> DNA
   <213> Artificial
<220>
   <223> Target
<400> 24
   tttcattctg cagcttcctc attt 24
<210> 25
   <211> 24
   <212> DNA
   <213> Artificial
<220>
   <223> Target
<400> 25
   tttcattctg caggttcctc attt 24
<210> 26
   <211> 24
   <212> DNA
   <213> Artificial
<220>
   <223> Target
<400> 26
   tttcattctg cagattcctc attt 24
<210> 27
   <211> 24
   <212> DNA
   <213> Artificial
<220>
   <223> Target
<400> 27
   tttcattctg cagtttcctc attt 24
<210> 28
   <211> 33
   <212> DNA
   <213> Artificial
<220>
   <223> Target
<400> 28
   tttagttttt ggacgtcttc tccttttcca att 33
<210> 29
   <211> 33
   <212> DNA
   <213> Artificial
<220>
   <223> Target
<400> 29
   tttagttttt ggacgtcttc tcccgatcga att 33
<210> 30
   <211> 33
   <212> DNA
   <213> Artificial
<220>
   <223> Target
<400> 30
   tttagttttt ggacgtcttc tccacagacg gtc 33
<210> 31
   <211> 33
   <212> DNA
   <213> Artificial
<220>
   <223> Target
<400> 31
   tttagttttt ggacgtcttc tccacagaca att 33
<210> 32
   <211> 33
   <212> DNA
   <213> Artificial
<220>
   <223> Target
<400> 32
   tttagttttt ggacgtcttc tccctttcca att 33
<210> 33
   <211> 33
   <212> DNA
   <213> Artificial
<220>
   <223> Target
<400> 33
   tttagttttt ggacgtcttc tccatttcca att 33
<210> 34
   <211> 33
   <212> DNA
   <213> Artificial
<220>
   <223> Target
<400> 34
   tttagttttt ggacgtcttc tcccgttcca att 33
<210> 35
   <211> 33
   <212> DNA
   <213> Artificial
<220>
   <223> Target
<400> 35
   tttagttttt ggacgtcttc tccacttcca att 33
<210> 36
   <211> 39
   <212> DNA
   <213> Artificial
<220>
   <223> Target
<400> 36
   tttcattctg cagcttcctc attgatggtc tcttttaac 39
<210> 37
   <211> 39
   <212> DNA
   <213> Artificial
<220>
   <223> Target
<400> 37
   tttcattctg cagcttcctc attgatggga tagtttaac 39
<210> 38
   <211> 39
   <212> DNA
   <213> Artificial
<220>
   <223> Target
<400> 38
   tttcattctg cagcttcctc attgatggag agatagggt 39
<210> 39
   <211> 39
   <212> DNA
   <213> Artificial
<220>
   <223> Target
<400> 39
   tttcattctg cagcttcctc attgatggag agatttaac 39
<210> 40
   <211> 39
   <212> DNA
   <213> Artificial
<220>
   <223> Target
<400> 40
   tttcattctg cagcttcctc attgatgggc tcttttaac 39
<210> 41
   <211> 39
   <212> DNA
   <213> Artificial
<220>
   <223> Target
<400> 41
   tttcattctg cagcttcctc attgatggac tcttttaac 39
<210> 42
   <211> 39
   <212> DNA
   <213> Artificial
<220>
   <223> Target
<400> 42
   tttcattctg cagcttcctc attgatggga tcttttaac 39
<210> 43
   <211> 39
   <212> DNA
   <213> Artificial
<220>
   <223> Target
<400> 43
   tttcattctg cagcttcctc attgatggag tcttttaac 39
<210> 44
   <211> 39
   <212> DNA
   <213> Artificial
<220>
   <223> Target
<400> 44
   ttgcagctct tctgccagtt tttggacgtc ttctccttt 39
<210> 45
   <211> 39
   <212> DNA
   <213> Artificial
<220>
   <223> Target
<400> 45
   ttgccgatcg tctgccagtt tttggacgtc ttctccttt 39
<210> 46
   <211> 39
   <212> DNA
   <213> Artificial
<220>
   <223> Target
<400> 46
   cagaagagga tctgccagtt tttggacgtc ttctccttt 39
<210> 47
   <211> 39
   <212> DNA
   <213> Artificial
<220>
   <223> Target
<400> 47
   ttgcagagga tctgccagtt tttggacgtc ttctccttt 39
<210> 48
   <211> 39
   <212> DNA
   <213> Artificial
<220>
   <223> Target
<400> 48
   ttgcagctcg tctgccagtt tttggacgtc ttctccttt 39
<210> 49
   <211> 39
   <212> DNA
   <213> Artificial
<220>
   <223> Target
<400> 49
   ttgcagctca tctgccagtt tttggacgtc ttctccttt 39
<210> 50
   <211> 44
   <212> DNA
   <213> Artificial
<220>
   <223> Probe
<220>
   <221> misc_feature
   <222> (1)..(10)
   <223> inverted nucleotides
<220>
   <221> misc_feature
   <222> (11)..(44)
   <223> natural nucleotides carrying (5'-3') phosphodiester linkages
<400> 50
   accctatctc tccatcaatg aggaagctgc agaatgggat agag 44